(19) Europäisches Patentamt
European Patent Office
Office européen des brevets

(11) EP 2 218 464 A1

(12) EUROPEAN PATENT APPLICATION

(43) Date of publication:
18.08.2010 Bulletin 2010/33

(21) Application number: 09001902.7

(22) Date of filing: 11.02.2009

(51) Int Cl.:
*A61K 51/04* (2006.01) *C07D 471/04* (2006.01)
*C07D 277/62* (2006.01) *C07D 513/04* (2006.01)
*C07D 487/04* (2006.01) *A61K 101/02* (2006.01)

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **Technische Universität München**
**81675 München (DE)**

(72) Inventors:
- **Henriksen, Gjermund**
  **80636 München (DE)**
- **Yousefi, Behrooz Hooshyar**
  **81738 München (DE)**
- **Wester, Hans-Jürgen**
  **85304 Ilmmünster (DE)**

(74) Representative: **Wichmann, Hendrik**
**Isenbruck Bösl Hörschler Wichmann LLP**
**Prinzregentenstrasse 68**
**81675 München (DE)**

# (54) Compounds for non-invasive measurement of aggregates of amyloid peptides

(57) The invention relates to the provision of compounds, methods for producing them, and their use for imaging and quantification of aggregates of amyloid peptides in vivo. In a preferred aspect of the invention, a tracer is administered to humans and displays enrichment in the areas that are containing amyloid plaques. Tracers of the invention can be used for in vivo visualization and quantification of aggregates of amyloid peptides in patients affected diseases characterized in the generation of aggregates of amyloid peptides, for example familial or sporadic Alzheimer's disease and type II diabetes. Tracers of the invention can be used for monitoring effects of amyloid-modulating therapies of patients affected with diseases characterized in the generation of aggregates of amyloid peptides, for example familial or sporadic Alzheimer's disease and type II diabetes.

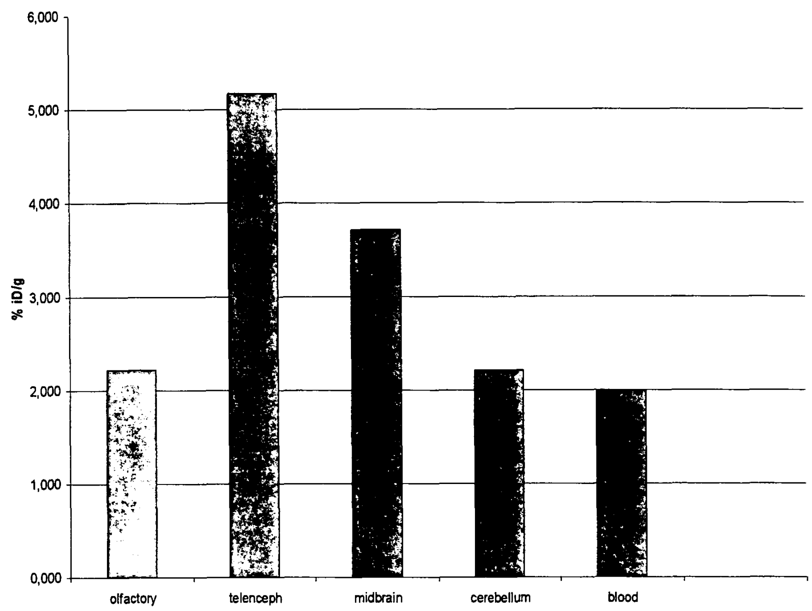


Fig. 8.

EP 2 218 464 A1

## Description

## BACKGROUND OF THE INVENTION

The prevalence of dementia, particularly Alzheimer's dementia (AD) is increasing with increasing life expectancy. Up to ~50% of persons > 85 years are suffering from AD, thus AD is one of the most common age-related disorders and is thus exerting major effects on socio-economic systems. Even though the causality of pathological processes underlying the development of dementias are not fully understood, growing evidence support the assumptions that a common basis of many neurodegenerative disorders can be found in increased production, misfolding and pathological aggregation of peptides or proteins.

The main constituents of Aβ-deposits in AD brain are peptides consisting of 40 and 42 amino acids, $A\beta_{40}$ and $A\beta_{42}$, generated from the cleavage of amyloid precursor protein. The subsequent arrangement of these peptide monomers includes amyloid fibrils, possessing a characteristic β-plated sheet structure, found in the amyloid core of neuritic plaques. Even though the physiological role of Aβ is not exactly clear, there is growing evidence for Aβ-peptides being essential for modulation of synaptic activity and neuronal survival under normal conditions (Pearson and Peers J Physiol. 2006;575:5-10). Based on familial cases, in which point mutations in the amyloid precursor protein is seen to increase amyloidosis, the formation and aggregation of amyloid peptides have been identified as critical factors in the initiation and progress of AD.

Above a certain critical concentration, Aβ40 and Aβ42 peptides apparently misfold and aggregate. $A\beta_{42}$ shows stronger aggregative tendency. Depending on density, configuration and constitution, different types of plaques have been described and in general, plaques with a lower proportion of fibrillar components (diffuse plaques) and a higher proportion of fibrillation (mature, dense core, classic or neuritic, or compact plaques) can be differentiated (Dickson and Vikers Neuroscience. 2001;105:99-107).

Recent studies indicate that not the entire Aβ plaques but possibly smaller Aβ oligomers may represent the responsible noxious species, leading to synaptic dysfunction. Several recent studies support LOF hypothesis, which implies that not just a general overproduction of Aβ is the initial trigger of the cascade of AD pathology but rather a imbalance between $A\beta_{40}$ and $A\beta_{42}$ amyloid peptides with a resulting relatively lower fraction of $A\beta_{40}$ (Van Broeck et al. Neurodegener Dis. 2007;4:349-65). It has been shown that $A\beta_{42}$ shows stronger aggregative tendency and preferentially assembles to noxious oligomers, representing the more toxic agents (Yan Y, Wang J Mol Biol. 2007;369: 909-16; Kim et al. J Neurosci. 2007;27:627-33). Some familiar forms of AD the level of $A\beta_{40}$ is decreased whereas the level of $A\beta_{42}$ remains stable. Recently, it has been discussed that $A\beta_{42}$ may actually exert an inhibitory function on presenilin function [Shen and and Kelleher Proc Natl Acad Sci U S A. 2007;104:403-9.

Aβ-induced neurotoxicity has been proposed to induce neurotoxicity by different mechanisms, including free radical generation (Hensley et al. Proc.Natl. Acad. Sci. USA 1994; 91: 3270-3274) oxidative stress (Yan et al. Nature 1996; 382: 685-69,115) or inflammation (Miklossy et al. J.Neuropathol. Exp. Neurol. 1999; 58: 803- 814). Biochemical and histopathological investigations have revealed that both early-onset familial AD and sporadic AD are characterized in an increased production of $A\beta_{42}$ (Citron et al. Nat Med 1997; 3:67-72; Mann et al. Ann Neurol 1996; 40:149-156; Kumar-Singh et al. Human mutation 2006; 27; 686-695, 2006) and up to two fold higher concentration of $A\beta_{42}$ relative to $A\beta_{40}$ in Aβ-aggregates (Kumar-Singh et al. Human mutation 2006; 27; 686-695). This has led to the $A\beta_{42}$ seed-hypothesis setting forth that $A\beta_{42}$ is the major driving force in providing Aβ-aggregates and thus neurotoxicity.

Historically, a definite diagnosis of AD has been obtained by neuropathological investigations of the brain from AD patients *post mortem*, depicting the presence of senile plaques containing aggregates of β-amyloid (Aβ) as well as neurofibrillary tangles consisting of highly phosphorylated tau protein. Current efforts to a vital method of AD diagnosis include determination of the presence of genes identified to represent an increased risk of developing the disease, determination of the level of amyloid peptides in the spinal fluid and in vivo imaging techniques.

New biomarkers such as for in vivo Aβ plaque imaging will open the potential to identify Alzheimer's disease (as a pathology which can be present in a person without symptoms) ahead of manifest Alzheimer dementia (as a clinical symptom complex). In addition to diagnosis, molecular imaging may play a relevant role in treatment selection and monitoring in the future. In AD, many modern treatment strategies are directed towards prevention or reversal of Aβ plaque deposition in the brain (e.g. β- and γ-secretase inhibitors, vaccination strategies) (Citron Nat Rev Neurosci. 2004; 5: 677-85). Amyloid plaque imaging may represent the most valuable surrogate marker particularly with regard to the well-known intraindividual fluctuations of cognitive performance.

An *in vivo* imaging technique could potentially be useful to assess the Aβ-load at a pre-symptomatic stage, to provide a differential diagnosis for AD over other neurodegenerative disorders and to monitor therapeutic effects directed at prevention or reversal of Aβ-plaque deposition in the brain.

In addition to the presence of aggregates of amyloid peptides in Alzheimer's disease, aggregates of amyloid peptides has been found in other diseases such as type II diabetes, Down's syndrome, Creutzfeldt-Jacob disease, prion mediated diseases, amyloid polyneuropathy and amyloid cardiomyopathy.

## SUMMARY OF THE INVENTION

The present invention provides compounds and tracers thereof for use as in vivo imaging probes in the depiction and quantification of aggregates of amyloid peptides. In a preferred embodiment, a tracer of the invention is administered to humans and is enriched in the aggregates of amyloid peptides, for example such contained in the brain of patients affected with AD, and is useful in discriminating between diseased individuals affected with aggregates of amyloid peptides and a normal individual. In another preferred aspect, radioactive analogues of the compounds of the invention can be obtained in a high yield and specific activity and have a low toxicity at an effective administered amount, including an amount being useful for imaging and quantification of aggregates of amyloid peptides or a disease state related to generation of aggregates of amyloid peptides. For example a tracer in the invention can be applied for depiction and quantification of aggregates of amyloid peptides, or aggregates of tau protein or synuclein. In yet another aspect of the invention compounds and radiolabeled tracers thereof can be applied for a preferential depiction and quantification of aggregates of amyloid peptides enriched in one or both of the peptide constituent, $A\beta_{40}$ and $A\beta_{42}$.

The present invention relates to provision of compounds that are binding to aggregates of amyloid peptides or intermediate stages of amyloid peptide polymers, parts or fragments of amyloid peptide polymers, in addition to aggregates constituted of amyloid peptides and other amyloidogenic peptide or protein. Tracers of the invention can be used to depict and quantify aggregates of amyloid peptides in individuals affected with diseases such as Alzheimer's disease, type II diabetes, Down's syndrome, Creutzfeldt-Jacob disease, prion mediated diseases, amyloid polyneuropathy and amyloid cardiomyopathy.

In one aspect, the invention relates to a tracer targeting aggregates of amyloid peptides in vitro or in vivo. The compounds and tracers of the invention can comprise the structures or formulas:

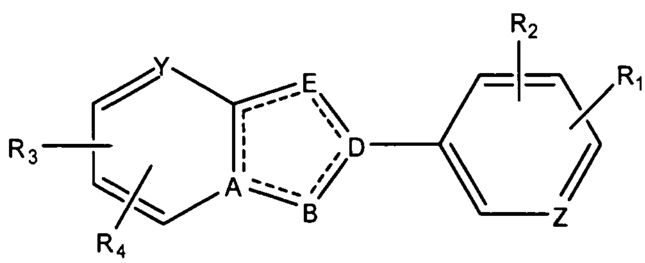

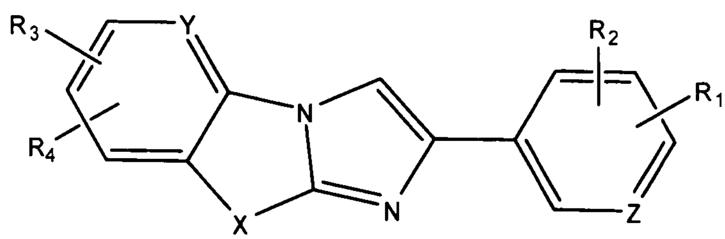

in which $R_1$, $R_2$, $R_3$ and $R_4$ are the same or different and can idependently be H, F, Cl, Br, I, CN, $CF_3$, alkyl, alkynyl, alkoxy, monoalkylamine, dialkylamine, hydroxyalkyl, haloalkyl, alkylthio, alkylsulfonyl, aryl, heterocycles, heteroaryl, carboxy, esterified carboxy, $OR_6$, $NR_5,R_6$ or $R_5,R_6$ can be $C_nH_{2n+1}$ or $-CH_2$-CH=CH-halo in which halo can be any halogen and $R_6$ can be $C_nH_{2n+1}$, $-[CH_2-CH_2-O]_m-R_5$ where n and m can independently be an integer in the interval 0-7, A and D can independently be N or C. E, Y and Z can independently be CH or N. B can be S, O N or CH. X can be N, S, or O. The compounds and tracers of invention can bind to and detect aggregates of amyloid peptides. In a preferred aspect of the invention, tracers of the invention can be constituted of a radionuclide including for example a photon emitting or a positron emitting radionuclide. For example a label can replace any substituent of a compound of the invention or be an additional substituent in a tracer. In one aspect, a tracer of the invention can comprise one or more of $^{3}H$, $^{11}C$, $^{18}F$, $^{123}I$, $^{124}I$, $^{75}Br$, $^{76}Br$.

In a preferred embodiment of the present invention, $R^1$ and $R^2$ are, independently of one another, substituted or unsubstituted, linear, branched or cyclic alkyl, alkenyl or alkynyl, optionally interrupted by one or more heteroatoms,

preferably substituted or unsubstituted, linear or branched $C_1$-$C_8$ alkyl, $C_2$-$C_8$ alkenyl and $C_2$-$C_8$ alkynyl, optionally interrupted by one or more heteroatoms, more preferably substituted or unsubstituted, linear or branched $C_1$-$C_6$ alkyl, $C_2$-$C_6$ alkenyl and $C_2$-$C_6$ alkynyl, optionally interrupted by one or more heteroatoms, and most preferably substituted or unsubstituted, linear or branched $C_1$-$C_4$ alkyl, $C_2$-$C_4$ alkenyl and $C_2$-$C_4$ alkynyl, optionally interrupted by one or more heteroatoms, or halogen. $R^1$ and $R^2$ may also be joined together to form a ring structure containing the nitrogen atom. The most preferred ring size contains 5 or 6 ring atoms. The most preferred substituents for $R_1$ and $R_2$ are H, methyl or ethyl or a cyclic, five-membered ring, or the halogens iod or brom.

**Table 1.**

| | |
|---|---|
| 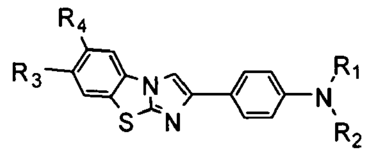 | $R_1$ = H, $C_nH_{2n}$, $C_nH_{2n+1}$,<br>$R_2$ = H, $C_nH_{2n}$, $C_nH_{2n+1}$<br>$R_4$ = H, $C_nH_{2n+1}$, F, Br, I or $OR_5$<br>$R_3$ = H, $C_nH_{2n+1}$, F, Br, I or $OR_5$ where $R_5$ = H, $C_nH_{2n+1}$, $C_nH_{2n}F$,<br>and n is an integer from 1-6 |
| 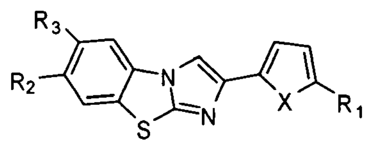 | X = N, S, O<br>$R_1$ = F, Br, I, Cl<br>$R_2$ = H, $C_nH_{2n+1}$, F, Br, I $OR_4$<br>$R_3$ = H, $C_nH_{2n+1}$, F, Br, I or $OR_4$ where $R_4$ = H, $C_nH_{2n+1}$ $C_nH_{2n}F$<br>and n is an integer from 1-6 |
| 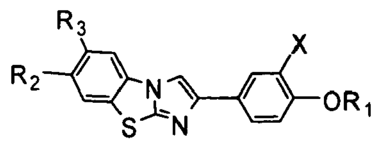 | X = F, Cl, Br, I<br>$R_1$ =H, $C_nH_{2n+1}$, $C_nH_{2n}F$<br>$R_2$ = H, $C_nH_{2n+1}$, F, Br, I $OR_4$<br>$R_3$ = H, $C_nH_{2n+1}$, F, Br, I or $OR_4$ where $R_4$ = H, $C_nH_{2n+1}$ $C_nH_{2n}F$<br>and n is an integer from 1-6 |
| 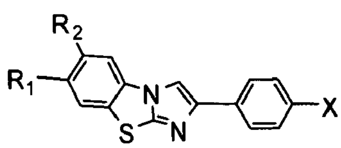 | X = F, Cl, Br, I<br>$R_1$= H, $C_nH_{2n+1}$, F, Br, I $OR_3$<br>$R_2$ = H, $C_nH_{2n+1}$, F, Br, I or $OR_3$ where $R_3$ = H, $C_nH_{2n+1}$ $C_nH_{2n}F$<br>and n is an integer from 1-6 |
| 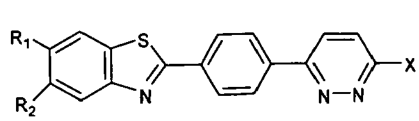 | X = F, Cl, Br, I<br>$R_1$= H, $C_nH_{2n+1}$, F, Br, I $OR_3$<br>$R_2$= H, $C_nH_{2n+1}$, F, Br, I or $OR_3$ where $R_3$ = H, $C_nH_{2n+1}$ $C_nH_{2n}F$<br>and n is an integer from 1-6 |
| 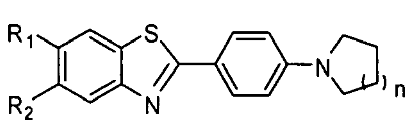 | $R_1$ = H, $C_nH_{2n+1}$, F, Br, I or $OR_3$<br>$R_2$ = H, $C_nH_{2n+1}$, F, Br, I or $OR_3$ where $R_3$ = H, $C_nH_{2n+1}$, $C_nH_{2n}F$.<br>and n is an integer from 1-3 |

(continued)

| Table 1. | |
|---|---|
| 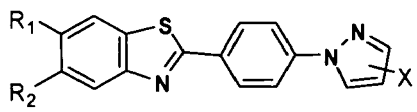 | X = F, Br, I<br>$R_1$ = H, $C_nH_{2n+1}$, F, Br, I or $OR_3$<br>$R_2$ = H, $C_nH_{2n+1}$, F, Br, I or $OR_3$ where $R_3$ = H, $C_nH_{2n+1}$, $C_nH_{2n}F$.<br>and n is an integer from 1-6 |
| 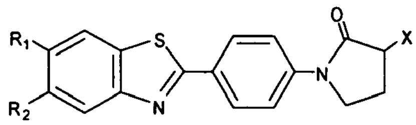 | X = F,Cl,Br,I<br>$R_1$ = H, $C_nH_{2n+1}$, F, Br, I or $OR_3$<br>$R_2$ = H, $C_nH_{2n+1}$, F, Br, I or $OR_3$ where $R_3$ = H, $C_nH_{2n+1}$, $C_nH_{2n}F$.<br>and n is an integer from 1-6 |
| 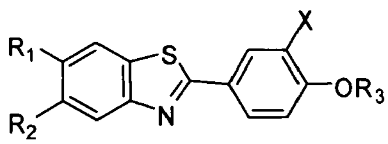 | X = H, F,Cl,Br, $NH_2$, O, $OR_3$<br>$R_1$ = H, $C_nH_{2n+1}$, F, Br, I or $OR_3$<br>$R_2$ = H, $C_nH_{2n+1}$, F, Br, I or $OR_3$ where $R_3$ = H, $C_nH_{2n+1}$, $C_nH_{2n}F$,<br>and n is an integer from 1-6 |
| 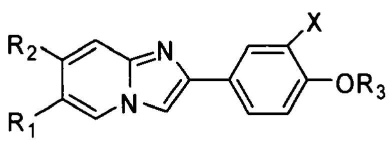 | X = H, F,Cl,Br, $NH_2$, O, $OR_3$<br>$R_1$ = H, $C_nH_{2n+1}$, F, Br, I or $OR_3$<br>$R_2$ = H, $C_nH_{2n+1}$, F, Br, I or $OR_3$ where $R_3$ = H, $C_nH_{2n+1}$, $C_nH_2nF$.<br>and n is an integer from 1-6 |
| 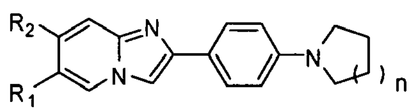 | $R_1$ = H, $C_nH_{2n+1}$, F, Br, I or $OR_3$<br>$R_2$ = H, $C_nH_{2n+1}$, F, Br, I or $OR_3$ where $R_3$ = H, $C_nH_{2n+1}$, $C_nH_{2n}F$.<br>and n is an integer from 1-3 |

The present invention also relates to preparation of compounds and tracers comprising a radionuclide, preferably a positron emitting or a photon emitting radionuclide, which upon administration to subjects can be used for depiction and quantification aggregates of amyloid peptides or a characteristic related to generation of amyloid aggregates. Examples of radionuclides include, but are not limited to, $^{11}C$, $^{18}F$, $^{123}I$, $^{124}I$, $^{75}Br$, $^{76}Br$.

In one aspect, the present invention relates to methods of radiolabeling for preparation of $^{18}F$-fluorinated compounds by direct fluorination. The compounds and tracers of the invention as they can comprise the structures or formulas

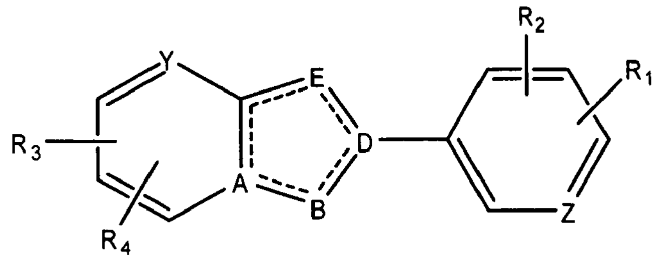

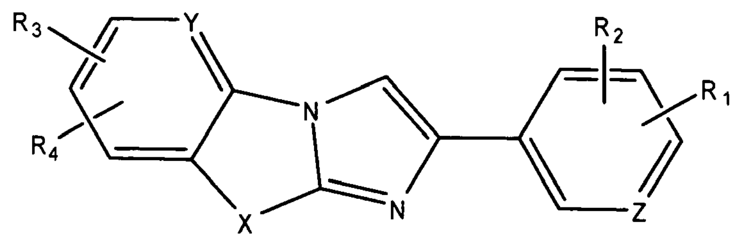

can made by substitution of a leaving-group-for-fluor.

In a precursor for radiosynthesis of a tracer of the invention, any suitable leaving group (LG) contained in one or more of $R_1$, $R_2$, $R_3$ or $R_4$ may be present as a group for the substitution reaction by fluorine. In a preferred embodiment LG is selected from the group consisting of halogen atoms, tosylsulfonate, trifluoromethanesulfonate, tolylsulfonatetriflate, tosylate, trialkyl ammonium, nitro and azide. Preferred halogen atoms are chlorine, bromine and iodine.

In a preferred embodiment of the present invention, the fluorination reaction is carried out in a solvent. The solvent is preferably a dry solvent in order to prevent the protonation of the fluoride anion. Aprotic dipolar or aprotic polar solvents are preferred as these solvent allow more easily for the solution of ionic atoms and molecules, especially the fluoride anion. Examples of solvents which may be used in the present application include dimethylformamide (DMF), tetrahydrofurane, (THF), dimethylsulfoxide (DMSO), acetonitrile, acetamide and dimethylacetamide (DMAA).

The reaction according to the present invention can be assisted by heating. Preferable combinations of solvent, heating temperature leaving group have been surprisingly found to allow for the preparation of $^{18}F$-labeled compounds by a direct fluorination method according to the present invention.

In one aspect, the present invention provides a method for fluorinating derivative. This fluorination can be applied to $^{18}F$-fluoride or any other fluoride isotope. The reaction itself does not differentiate between different isotopes. As fluoride is always present within any solvent, the concentration of the $^{18}F$-fluoride of interest is measured by its radioactivity. When expressing the ratio of the radioactive fluoride in relation to the aniline derivative, the ratio of the $^{18}F$-fluoride to the aniline derivative is in the range of 1:1,000-100,000, preferably in the range of 1:1,000-10,000 and more preferably in the range of 1:2,000-5,000.

According to another aspect of the invention, the invention relates to the use of tracers of the invention in non-invasive imaging, for instance by means of positron emission tomography (PET) and/or single photon emission tomography (SPECT) and to the use of such compounds in clinical studies in humans and other mammals.

More preferably, the present invention relates to the use of a compound belonging to the invention in the imaging of aggregates of amyloid peptides in the central nervous system. The production and presence of one or more pathological proteins, especially amyloid plaques, is encountered in several human diseases and syndromes, including Alzheimer's disease, mild cognitive impairment syndrome, Parkinson's disease, Lewy body dementia, Down's syndrome, frontotemporal degeneration type II diabetes, Creutzfeldt-Jacob disease, prion mediated diseases, amyloid polyneuropathy and amyloid cardiomyopathy.

A method of the invention can also include the determination of a localization index of a tracer. In one aspect, the localization index can be the uptake of the tracer in a brain region of interest other than the cerebellum relative to that of cerebellum. In another aspect, the localization index can comprise a comparison of the amount of tracer located in the region of interest of a subject affected with, or suspected to be affected with, a disease related to aggregation of amyloid peptides relative to that obtained in a normal control.

## DESCRIPTIONS OF THE DRAWINGS

**FIG 1**. Transmission electron microscopy (TEM) images of amyloid aggregates fixed using a negative staining material, uranyl acetate. A and B are TEM images of $A\beta_{40}$ of 33000 and 50000 times magnification, respectively; C and D are TEM images of $A\beta_{42}$ of 33000 and 50000 times magnification, respectively.

**FIG 2.** Inhibition of binding of *N*-[$^3$H-methyl]6-OH-BTA-1 to fibrils of Aβ40 and Aβ42 from test compounds present at 100 nM.

**FIG 3:** $K_i$ values (nM) determined for inhibition of *N*-[$^3$H-methyl]6-OH-BTA-1 binding to β-amyloid fibrils.

**FIG 4.** Inhibition of binding of *N*-[$^3$H-methyl]6-OH-BTA-1 to fibrils of Aβ40 and Aβ42 from test compounds present at 100 nM.

**FIG 5.** Brain uptake of selected compounds of the invention and [$^{11}$C]PIB in male Balb-C mice at 5 and 30 min post injection.

**FIG 6.** Log P values for selected compounds of the invention.

**FIG. 7.** Speciation of radioactivity in brain and blood of mice injected with [$^{11}$C]**8** or [$^{18}$F]**15.**

**FIG. 8** Regional ex vivo brain biodistribution of [$^{11}$C]**8** in a PS1/APP double transgenic mouse model of Alzheimer's disease.

**FIG. 9.** Staining of brain sections of a PS1/APP double transgenic mouse model of Alzheimer's disease.with Thioflavin-T (left) and fluorescent antibodies (right).

**FIG.10.** Summed PET-images (20-35 min post injection) of [$^{11}$C]8 in a PS1/APP double transgenic mouse model of Alzheimer's disease of age 16 months (A) and in an age-matched control animal (B).

**FIG. 11.** Summed PET-images (20-35 min post injection) in a PS1/APP double transgenic mouse model of Alzheimer's disease of age 15.6 months injected with [$^{11}$C]**8** (A) or [$^{18}$F]**15** (B).

**FIG.12.** *Left:* Ex vivo autoradiography of an axial section of the brain of a PS1/APP double transgenic mouse model of Alzheimer's disease killed 40 min after injection of [$^{11}$C]**8**. *Right:* Profile of the activity distribution on the section demonstrating enrichment of activity in plaque containing areas.

**FIG. 13.** *Left:* Ex vivo autoradiography of an axial section of the brain of an PS1/APP brain killed 40 min after injection of [$^{18}$F]**15**. *Right:* Profile of the activity distribution on the section demonstrating enrichment of activity in plaque containing areas.

**FIG.** 14. Immunohistochemistry (A) and digital autoradiography of *N*-[$^3$H-methyl]6-OH-BTA-1 binding to human AD-brain sections incubated *in vitro* (B) without (i) and with (ii) the presence of compound **8** (left) or compound **15** (right).

**FIG. 15.** Ex vivo dual tracer autoradiography of a coronal brain section (right hemisphere) of the brain of an PS1/APP mice brain killed 30 min. after injection of a mixture of [$^{11}$C]8 and *N*-[$^3$H-methyl]6-OH-BTA-1. The raw data generated in the dual tracer mode and was reconstructed to generate the contribution from the respective radionuclides based on the differences in half-life by means of the beta vision software. The distribution of radioactivity is fused with the optical image. A: Separated image of [$^{11}$C]**8**. B: Separated image of *N*-[$^3$H-methyl]6-OH-BTA-1.

**FIG. 16.** Brain uptake of selected compounds of the invention and *N*-[$^3$H-methyl]6-OH-BTA-1 in male Balb-C mice at 5 and 30 min post injection.

## DETAILED DESCRIPTION OF THE INVENTION

The present invention provides compounds, tracers thereof for use as in vivo imaging probes in the depiction

and quantification of aggregates of amyloid peptides, for example amyloid plaques. In one aspect of the invention a tracer of the invention is administered to humans and is enriched in the aggregates of amyloid peptides, for example such contained in the brain of patients affected with AD, and is useful in discriminating between diseased individuals affected with aggregates of amyloid peptides and a normal individual. In another aspect, radioactive analogues of the compounds of the invention can be obtained in a high yield and specific activity and have a low toxicity at an effective administered amount, including an amount being useful for imaging and quantification of aggregates of amyloid peptides or a disease state related to generation of aggregates of amyloid peptides. Tracers of the invention can be used to depict and quantify aggregates of amyloid peptides in individuals affected with diseases such as Alzheimer's disease, mild cognitive impairment syndrome, Parkinson's disease, Lewy body dementia, Down's syndrome, frontotemporal degeneration. type II diabetes, Creutzfeldt-Jacob disease, prion mediated diseases, amyloid polyneuropathy and amyloid cardiomyopathy.

In one aspect the invention relates to a tracer targeting aggregates of amyloid peptides in vitro or in vivo. The compounds and tracers of the invention can comprise the structure or formula:

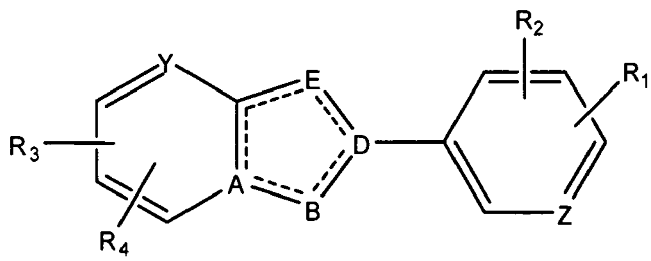

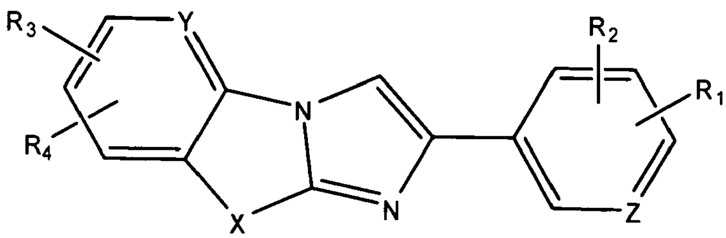

in which $R_1$, $R_2$, $R_3$ and $R_4$ are the same or different and can independently be H, F, Cl, Br, I, CN, $CF_3$, alkyl, alkynyl, alkoxy, monoalkylamine, dialkylamine, hydroxyalkyl, haloalkyl, alkylthio, alkylsulfonyl, aryl, heterocycles, heteroaryl, carboxy, esterified carboxy, $OR_6$, $NR_5$,$R_6$ or $R_5$,$R_6$ can be $C_nH_{2n+1}$ or $-CH_2-CH=CH-$halo in which halo can be any halogen and $R_6$ can be $C_nH_{2n+1}$, $-[CH_2-CH_2-O]_m-R_5$ where n and m can independently be an integer in the interval 0-7, A and D can independently be N or C. E, Y and Z can independently be CH or N. B can be S, O N or CH. X can be N, S, or O, and the tracers of invention can bind to and detect aggregates of amyloid peptides. In a preferred aspect, tracers of the invention can be constituted of a radionuclide including for example a photon emitting or a positron emitting radionuclide. For example a label can replace any substituent of a compound of the invention or be an additional substituent in a tracer. In one aspect, a tracer of the invention can comprise one or more of $^{3}H$, $^{11}C$, $^{18}F$, $^{123}I$, $^{124}I$, $^{75}Br$, $^{76}Br$.

In one aspect, compounds and tracers of the invention can be used for detection of aggregates of amyloid peptides in the body by means of PET. In another aspect, compounds and tracers of the invention can be used for detection of aggregates of amyloid peptides in the body by means of SPECT. In yet another aspect, given appropriate configuration and constitution of the molecule, the compounds and tracers of the invention can be used for detection of aggregates of amyloid peptides in the body by means of PET and SPECT. The compounds and tracers of the invention can be fitted, according to demand, with one or both of a positron emitting radionuclide, useful for PET, and a photon emitting radionuclide, useful for PET. In such a manner, an identical chemical compound can be used for PET and SPECT depending on the radionuclide in question implemented.

A method of the invention can be applied to prepare tracers preferably detectable in the body by use of PET and/or SPECT. A compound or tracer of the invention can contain radiohalogens. The radiohalogen can preferably be

selected among fluorine, iodine and bromine radioisotopes. For SPECT, the halogens of suitable half-life and gamma energy for biomedical applications include without limitation, $^{123}I$ (t ½ = 13 h, Eγ 159 keV), $^{123}I$ (t ½ = 8.3 d), and $^{77}Br$, $^{75}Br$ and $^{76}Br$. Bromine and iodine radionuclides can be introduced into a compound of the invention according to methods known in the art (Wilbur. Bioconjug Chem. 1992;3:433-70; Maziere et al. Radioionidation Reactions for Pharmaceuticals, Springer Verlag, Berlin).

[$^{18}F$]Fluoride is one of the most useful PET radionuclides currently being used in clinical nuclear medicine diagnosis. With regard to the nuclear properties, $^{18}F$ has a relatively pure (97 % probability) decay mode through the emission of a positron ($\beta^+$) of which the $E_{\beta+max}$ of 633.5 keV is the lowest of the radionuclides that are commonly used in PET. Consequently, the average range of the positron from $^{18}F$ in biological tissues of about 0.3 mm is less than the inherent resolution of clinical PET-scanners. The 109.7 min half-life of $^{18}F$ allows for the production and distribution of radiotracers to nuclear medicine facilities without an on-site cyclotron facility and scanning procedures extending several hours.

[$^{18}F$]Fluoride is commonly available from cyclotrons on a Ci-scale (1 Ci = 37 GBq). Among the four main production routes that have been described in the literature, the $^{18}O(p,n)^{18}F$ nuclear reaction on highly enriched $H_2[^{18}O]O$ is the most effective one, and gives [$^{18}F$]Fluoride in high specific activities (up to 5000 mCi/μmol) suitable for nucleophilic reactions of a variety of substrates.

$^{18}F$-labeled compounds can be used in positron emission tomography (PET) to allow for the non-invasive imaging of, *inter alia*, biological structures, particularly those of mammals. This can involve depiction and quantification of receptors and transporters. To be generally useful, a radiopharmaceutical based on a short lived radionuclide such as $^{18}F$ needs to be obtainable rapidly in high radiochemical yield and with high specific radioactivity. $^{18}F$ has relatively short physical half life of 109.7 min, which means that any process for the manufacture of $^{18}F$-labeled compounds needs to occur in a short time period and has to be capable of providing the desired yields and specific activity of the compounds in question.

The literature comprises many different ways of forming $^{18}F$-labeled compounds although many of these processes involve the use of a precursor for radiolabeling activated for nucleophilic attack. The literature comprises some ways of preparing $^{18}F$-labeled compounds by electrophilic substitution. For preparing $^{18}F$-labeled compounds in a high specific radioactivity (>100 mCi/μmol), this method is hampered by the necessity of introducing carrier fluorine.

The vast majority of fluorination syntheses described in the literature concern the formation of non-radioactive species and the chemistry involved is therefore not applicable as outlined below. The process requirements for the preparation of non-radioactive fluorine compounds are undemanding compared to synthesis of radioactive compounds.

High radiochemical yield is obviously desirable in the same way that high yield is desirable in any organic synthesis. If more $^{18}F$ is incorporated into the final product then the amount of active compound formed is higher and there is less wasted expensive starting material.

High specific radioactivity is an important issue in PET studies where the amount of tracer should be minimized to keep the biological system unperturbed. Especially for compounds intended for use in quantifying receptors in the central nervous system (CNS), which is often present at a relatively low concentration, these requirements are often limiting. Thus, these requirements impose significant limitations on the chemical-synthetic procedures used for the preparation of the $^{18}F$-labeled compounds relative to non-radioactive compounds.

A further problem faced during the synthesis of $^{18}F$-labeled compounds is the possibility of side reactions. In conventional non radioactive synthesis, stoichiometric amounts of reactants would be used. This is often not possible in $^{18}F$-compound synthesis and there is typically considerable molar excess of reagents and reactants than the $^{18}F$-labeled species. The difference in the molar ratio of the $^{18}F$-containing reagent relative to other reagents present in the reaction mixture (including the molecule or species to be radiolabeled, called the precursor for radiolabeling) can represent an important difference to procedures in which the stoichiometry of reagents is in the range normally encountered in the synthesis of non-radioactive compounds. In particular, due to the relatively low amount of $^{18}F$-containing reactant present, impurities in the reaction mixture can be an important loss of yield if they possess a comparable or higher reactivity than the precursor for radiolabeling. Thus, side-reactions that are of low importance for a high yield of a given product in a non-radioactive synthesis can be a limiting factor for $^{18}F$-radiolabeling reactions.

A further issue with $^{18}F$ syntheses is the potential exposure of the chemist to radiation. $^{18}F$ syntheses of radiopharmaceuticals for use in PET are typically performed in lead shielded boxes (called hot-cells) and the manipulation of reagents, reactants and equipment is performed via a remote controlled apparatus (called the syntheses module). Under such conditions, the number of synthetic steps, the overall length of the procedure performed with the radioactivity present greatly affects the performance, including reliability and reproducibility, of the procedure. It is therefore advantageous to have as few steps in the procedure as possible, with the highest possible radiochemical yield in order to ensure a high performance of the radiosynthesis and to reduce the complexity of the apparatus required.

In nucleophilic fluorination starting with no-carrier-added, [$^{18}F$]fluoride is the sole suitable approach for preparing high specific activity $^{18}F$-labeled compounds. Due to its high charge density, the fluoride ion is highly solvated in aqueous solution and a relatively poor nucleophile. As a strong base, it is protonated in the presence of acidic protons to form

$H[^{18}F]F$. Consequently, several major aspects have to be considered in order to reach a nucleophilic $^{18}F$-fluorination that is suitable for preparative procedure in radiopharmaceutical chemistry. Firstly, the fluoride ion has to be activated in order to achieve a useful nucleophilicity and solubility. Often dipolar aprotic solvents are used as the reaction medium in order to avoid protonation of the fluoride. Due to the low molar concentration of reactive $^{18}F$-fluoride encountered at labeling reactions designed for obtaining a high specific activity of the product, potential undesired processes such as absorption losses, chemical side reactions and pseudo-carrier effects and presence of water implies that high chemical purity of chemicals, precursors and solvents are required to obtain a relevant radiochemical yield.

An object of the present invention is to provide a fast method for the preparation of $^{18}F$-labeled aniline derivatives wherein the $^{18}F$ is introduced in a single step.

A $^{18}F$-labeled is a compound comprising the linkage -C-$^{18}F$ where C is a *sp*$^{3}$ hybridized carbon atom. The $^{18}F$ is introduced into a molecule containing a leaving group. The skilled chemist is aware of numerous leaving groups regularly used in organic synthesis and any of these may be used here. Preferred leaving groups are listed in standard text books such as Jerry March, Advanced Organic Chemistry and include alkoxys, halides and sulphonate esters. Preferred halides are chloride, bromide and iodide. Preferred sulphonate esters are brosylate, nosylate, mesylate, nonaflate, tresylate, triflate or tosylate, especially triflate, tosylate or mesylate. Halides and sulphonate esters are preferred in particular sulphonate esters of low molecular mass. Preferably therefore X is iodide, triflate or tosylate, especially tosylate.

The leaving group is preferably bound to a *sp*$^{3}$ hybridized carbon atom being a part of a molecule containing the group -O-$R^1$ or N-$R^1$,$R^2$, where $R^1$ and $R^2$ are an alkyl group, alkylene group or hydrogen. Thus, R can also contain many other functional groups such as aryl, cycloalkyl, heterocyclic groups as well as functionalities such as hydroxyl, and other non nucleophilic and non electrophilic groups. R can therefore vary considerably as long as other functional groups present therein do not interfere with the desired C-$^{18}F$ formation reaction.

Yields of greater than 40%, e.g. greater than 50% can also be achieved. The reactions described herein are preferably effected with no carrier added $^{18}F$.

The [$^{18}F$]fluoride is present in the reaction as an anion. In order to bring this [$^{18}F$]fluoride into a solution, a corresponding cation also has to be present. This cation is preferably a phase transfer catalyst. Any known phase transfer catalyst can be used. Preferred cationic phase transfer catalysts are tetraalkyl ammonium salts.

In an embodiment of the present application, a tetrabutyl ammonium [$^{18}F$]fluoride can be used. Other ammonium salts include tetramethyl ammonium [$^{18}F$]fluoride, tetraethyl ammonium floride, tetra(n-propyl) ammonium floride and tetra(iso-propyl) ammonium $^{18}F$-fluoride. The tetrabutyl ammonium [$^{18}F$]fluoride may be any tetrabutyl ammonium [$^{18}F$] fluoride, i.e. the butyl may be n-butyl, iso-butyl or *tert*.-butyl. It will be obvious to a person skilled in the art that any tetraalkyl ammonium salt may be used. This encompasses also ammonium salts wherein different alkyl radicals are present, e.g. dimethyl-diethyl ammonium [$^{18}F$]fluoride. However, other tetraalkyl ammonium salts may also be used, including salts wherein the alkyl groups are linear, branched or cyclic alkyl radicals. The above given definition of alkyl may also apply to the alkyl radicals of the tetraalkyl ammonium salts.

Other feasible kationic phase transfer include crown ethers and cryptants. Preferably, a cryptand known as cryptand [2.2.2]. The most preferred cryptands are K $[2.2.2]^+$ and Cs $[2.2.2]^+$.

The solution containing the [$^{18}F$]fluoride may preferably contain further anions to stabilize the solution. By stabilization, a constant pH value is meant. The pH of the solution should be kept in the basic region in order to prevent the [$^{18}F$]fluoride from being protonated. The protonated form HF of the [$^{18}F$]fluoride can not be used for the method according to the present application. To prevent this protonation, the pH of the solution should be higher than 7. This can be achieved by e.g. addition of basic anions. In a preferred embodiment, the solution of the [$^{18}F$]fluoride according to the present invention contains carbonate anions. Another preferred anionic pH stabilizing anion is oxalate. Several pH stabilizing anions may also be used at the same time.

Another object of the present invention is to provide $^{18}F$-labled derivatives which can be used for PET diagnostics.

Still another object of the present invention is to provide a diagnostic method for the diagnosis of amyloid related diseases.

The objects of the present invention are achieved by the independent claims. Preferred embodiments are set forth in the dependent claims.

In one aspect the invention relates to preparation of a compound or tracer comprising the structure

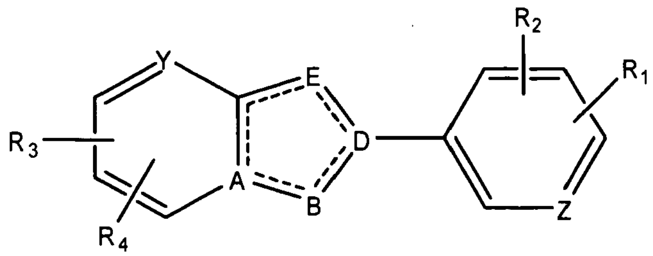

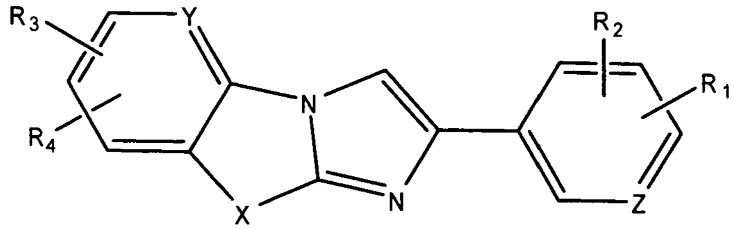

where one or more of $R_1$, $R_2$ $R_3$, $R_4$ contains a fluor atom bound to an alkyl chain. The term alkyl as used within this application includes any linear, branched or cyclic saturated hydrocarbon. Preferably, the alkyl is a $C_1$ to $C_{10}$ alkyl, more preferably a $C_1$ to $C_8$ alkyl and even more preferably a $C_1$ to $C_6$ alkyl. However, this carbon chain may optionally be substituted or interrupted by heteroatoms like N, O and S. Preferred are interruptions by up to four heteroatoms within the carbon chain. Examples of these interruptions include ethers, thioethers, amines and polyethylene glycols (PEGs). Preferred aliphatic chains with heteroatom interruptions include PEG with 2 to 6 PEG groups and their corresponding polythioether equivalents. In another preferred embodiment, the PEG substituents contain a halogen substitution, preferably a terminal halogen substitution.

Optionally, the alkyl may further be substituted. Substitution include halogen atoms, groups containing heteroatoms like OR, $NR_2$, SR, SCN, CN, COOR, wherein R is H, alkyl, alkenyl, alkynyl or aryl.

The term "alkenyl" can refer to an unsaturated straight of branched chain hydrocarbon radical comprising at least one carbon-to-carbon double bond. The chain can be constituted of up to 8 carbons, preferably 5 carbons, more preferably 4 carbons. Examples include, but are not limited to ethenyl, propenyl, iso-propenyl, butenyl, isobutenyl, t-butenyl. Furthermore, "alkenyl" can refer to an unsaturated straight or branched chain hydrocarbon radical comprising at least one carbon-to-carbon triple bound. The chain can be constituted of up to 8 carbons, preferably 5 carbons, more preferably 4 carbons. Examples include, but are not limited to ethylyl, propynyl, iso-propynyl, butynyl, isobutynyl, t-butynyl. A compound or tracer of the invention can comprise one or more alkenyl or alkynyl substituents.

The term "alkoxy" can refer to a straight or branched chain alkyl radical bonded to an oxygen atom of a chain length of 1-8 carbons, preferably 1-6 carbons, more preferably 1-4 carbons. Examples include, but are not limited to, methoxy, ethxoy, n-propoxy, isopropoxy, butoxy, iso-butoxy, t-butoxy. A compound or tracer of the invention can comprise one or more alkoxy substituents.

The term "monoalkylamine" by itself or as a part of another group can refer to an amino group that is substituted with one alkyl group. In one aspect, the term "methylamino" can refer to a neutral group or ring substituent in which N is connected to a compound of the invention via the ring or a chain of the compound and N is further bound to a methyl and a hydrogen. The N may be charged and form a salt. A compound or tracer of the invention can comprise one or more monoalkylamine substituents.

The term "dialkylamine" by itself or as a part of another group can refer to an amino group that is substituted with two alkyl groups. In one aspect, the term "dimethylamino" can refer to a neutral group or ring substituent in which N is connected to a compound of the invention via the ring or a chain of the compound and N is further bound to two methyl groups. The N may be charged and form a salt. A compound or tracer of the invention can comprise one or more dialkylamine substituents.

The term "hydroxy($C_{1-5}$)alkyl" can refer to an alkyl chain connected to a compound of the invention via the ring or chain of the compound in which the distal portion of the alkyl chain of the group contains a hydroxy moiety. Preferably, the number of carbons in the alkyl chain is from 1 to 5. A compound or tracer of the invention can comprise one or more

hydroxy($C_{1-5}$)alkyl substituents.

The term "halo" or "halogen" by itself or as a part of another group can refer to fluorine, chlorine, bromine or iodine. A compound or tracer of the invention can comprise one or more halo substituents.

The term "haloalkyl" can refer to any of the mentioned alkyl groups substituted by one or more fluorine, chlorine, bromine or iodine such as chloromethyl, iodomethyl, trifluoromethyl, 2,2,2-trifluoroethyl and 2-chloroethyl. A compound or tracer of the invention can comprise one or more haloalkylsubstituents.

The term "alkylthio" by itself or as a part of another group can refer to a thioether of the structure R*-S in which R* is a $C_{1-4}$ alkyl. A compound or tracer of the invention can comprise one or more alkylthio substituents.

The term "heterocycle" or "heterocyclic ring" can represent a 4 to 7-membered mono-heterocyclic ring that may be saturated or unsaturated, and consist of carbon atoms and from one to three heteroatoms selected from the group consisting of N, O and S. Examples include, but are not limited to piperidinyl, pyrrolyl, pyrrolidinyl, imidazolyl, imidazinyl, imidazolinyl, pyridyl, pyrazinyl, pyriminidyl, oxaxzolyl, oxazolidinyl, isoxazolyl, isoxazolindinyl, thiazolyl, thiazolidinyl, isothiazolyl, homopiperidinyl, homopiperazinyl, pyridazinyl, pyrazolyl, pyrazolidinyl. A compound or tracer of the invention can comprise one or more heterocycle or heterocyclic ring substituents.

The term "heteroatom" can represent an oxygen atom ("O"), a sulphur atom ("S") or a nitrogen atom ("N"). It will be recognized that when the heteroatom is nitrogen, it may form an NR'R" in which R' and R" are, independent from another, hydrogen, $C_{1-4}$ alkyl, $C_{2-4}$ aminoalkyl, $C_{1-4}$ haloalkyl or halo benzyl. R' and R" can be taken together to form a 5 to 7-member heterocyclic ring that optionally comprises O, S or NR"' in which R"' is hydrogen or $C_{1-4}$ alkyl. A compound or tracer of the invention can comprise one or more heteroatom substituents.

**Example 1**

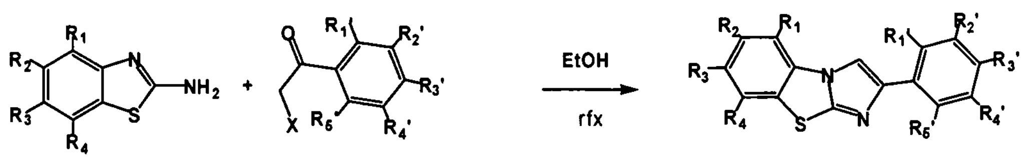


1 2 3

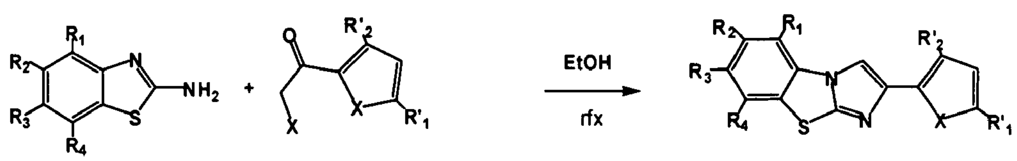


1 2' 3'

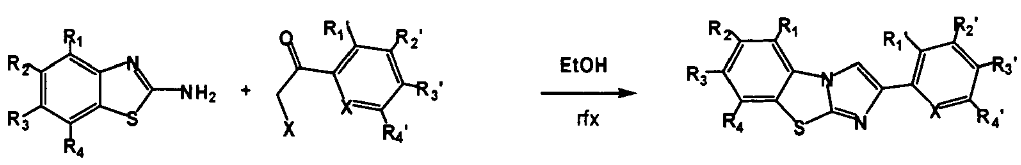


1 2" 3"

Synthesis of a set of compounds.

2-Phenylimidazo[2,1-*b*]benzothiazoles **3** were prepared from the appropriately substituted 2-aminobenzothiazoles **1** and the appropriately substituted phenacylhalides **2**. Treatment of **1** with equimolar amount of **2** in ethanol at

refluxing temperature afforded **3** in moderate to excellent yields (37-95%). All the products and intermediates were characterized by mass spectrometry and their purities checked by HPLC. Selected compounds were also confirmed by $^{1}H$-NMR and $^{13}C$-NMR.

Similarly, 2-heteroaryl imidazo[2,1-*b*]benzothiazoles were prepared from the appropriately substituted 2-aminobenzothiazoles **1** and the appropriately substituted 2-bromo-1-(heteroaryl)ethanones (**2**' or **2**").

4-(7-Methoxyimidazo[2,1-*b*]benzothiazol-2-yl)-*N*,*N*-diethyl-benzenamine (**1**).

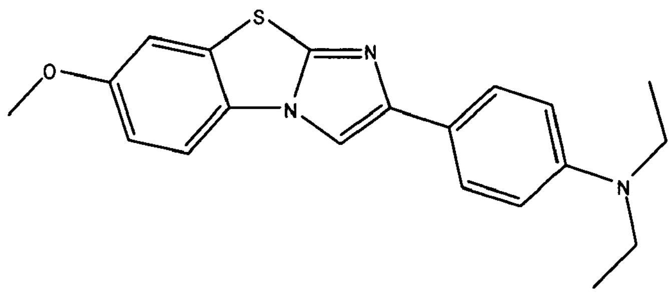

A mixture of 6-methoxybenzo[*d*]thiazol-2-amine (180 mg, 1 mmol) and 2-bromo-1-(4-(diethylamino)phenyl) ethanone (270 mg, 1 mmol) in 5 ml EtOH was heated overnight at reflux..The reaction mixture was cooled to room temperature, filtered, washed with 2 ml ether and the precipitate, 4-(7-methoxyimidazo[2,1-*b*]benzothiazol-2-yl)-*N*,*N*-diethyl-benzenamine, was dried under vacuum (263 mg, 75% yield, > 95% as measured by.HPLC purity). ESI-MS , [M+1] =352.1; DMSO-$d_6$ $^{1}H$ NMR $\delta$ 1.07 (6H,t), 3.63 (4H,q), 3.83 (3H,s), 7.18 (2H,d), 7.72 (2H,s), 7.93 (2H, d), 8.80 (1H,s), 11.22 (1H,s); $^{13}C$ NMR $\delta$ 11.3, 56.8, 100.0, 102.2, 108.2, 114.9, 115.0, 126.6, 126.9, 131.4, 148.9, 158.0.

**Example 2.** 4-(7-Hydroxyimidazo[2,1-*b*]benzothiazol-2-yl)-*N*,*N*-diethyl-benzenamine (**2**).

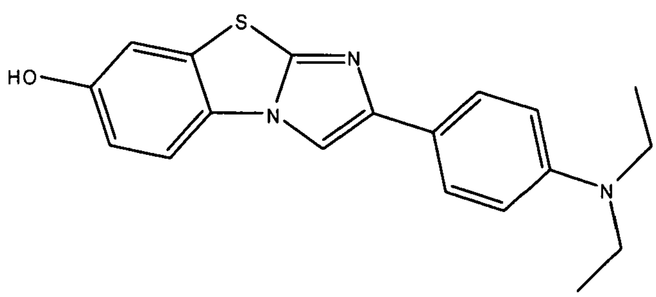

Compound **1** (105 mg, 0.3 mmol) was reacted with 1.5 molar equivalents of $BBr_3$ (1 molar solution) in 10 ml dichloromethane under microwave heating at 150˚C for 20 min.. The reaction mixture was quenched with 10 ml water and extracted with 2 × 10 ml $CH_2Cl_2$. The combined organic phase was washed with saturated Sodium bicarbonate solution (10 ml), dried over sodium sulphate and evaporated. The crude product, **2**, 4-(7-hydroxyimidazo[2,1-*b*]benzothiazol-2-yl)-*N*,*N*-diethyl-benzenamine, was recrystallized from MeOH (92 mg, 91% yield) and used for further $^{11}C$-methylation reaction. ESI-MS, [M+1]= 338.1; DMSO-$d_6$ $^{1}H$ NMR $\delta$ 1.10 (6H,t), 3.35 (4H,q), 6.69 (2H,d), 6.92 (1H,d), 7.35 (1H,s), 7.62 (2H,d), 7.73 (1H,d), 8.35 (1H,s), 9.83 (1H,s); $^{13}C$ NMR $\delta$ 11.4, 44.5, 107.1, 111.6, 112.4, 114.4, 115.1, 122.1, 125.9, 126.7, 131.0, 146.2, 147.5, 147.6, 155.7.

As an alternative procedure, **2** was also prepared from **1** by using 3 equivalent of 1 molar solution of $BBr_3$ in dichloromethane at room temperature during 48 h.

**Example 3.** 4-(7-Methoxyimidazo[2,1-*b*]benzothiazol-2-yl)-*N*,*N*-dimethyl-benzenamine (**3**).

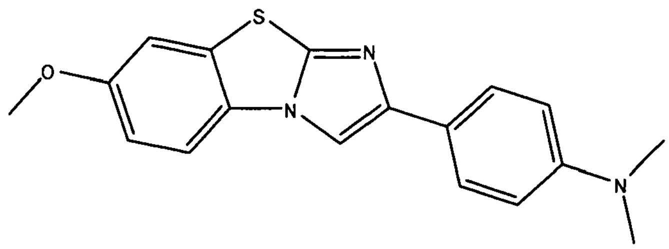

A mixture of 6-methoxybenzo[*d*]thiazol-2-amine (180 mg, 1 mmol) and 2-bromo-1-(4-(dimethylamino)phenyl) ethanone (242 mg, 1 mmol) in 5 ml EtOH was heated overnight at reflux. The reaction mixture was cooled to room temperature, filtered, and washed with 2 ml ether and the resulting precipitate, 4-(7-methoxyimidazo[2,1-*b*]benzothiazol-2-yl)-*N*,*N*-dimethyl-benzenamine, dried under vacuum (246 mg, 76% yield, purity > 95% as measured by HPLC ESI-MS, [M+1]=324.1; DMSO-$d_6$ $^1$H NMR $\delta$ 3.06 (6H,s), 3.85 (3H,s), 6.55 (2H,d), 7.38 (2H,d), 7.72 (3H,m), 7.98 (2H, d), 8.80 (1H,s); $^{13}$C NMR $\delta$ 43.0, 56.8, 108.3, 109.4, 110.4, 111.6, 113.0, 115.3, 126.5, 126.7, 131.4, 146.1, 158.3.

**Example 4.** 4-(7-hydroxyimidazo[2,1-*b*]benzothiazol-2-yl)-*N*,*N*-dimethyl-benzenamine (**4**).

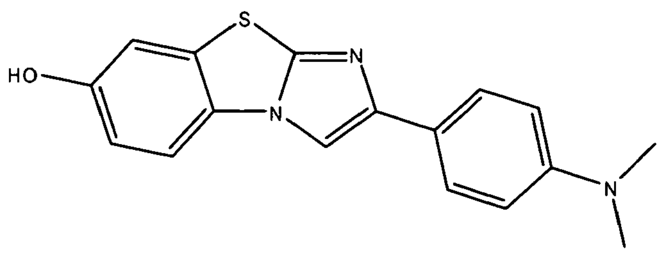

Compound **3** (97 mg, 0.3 mmol) was reacted with 1.5 molar equivalents of $BBr_3$ (1 molar solution) in 10 ml dichloromethane under microwave heating at 150˚C for 20 min. The reaction mixture was quenched with 10 ml water and extracted with 2 × 10 ml $CH_2Cl_2$. The combined organic phase was washed with saturated Sodium bicarbonate solution (10 ml), dried over sodium sulphate and evaporated. The crude product, 4-(7-hydroxyimidazo[2,1-*b*]benzothiazol-2-yl)-N,N-dimethyl-benzenamine, **4**, was recrystallized from MeOH (87 mg, 90% yield) and used further for $^{11}$C-methylation reaction. LC-MS-ESI, [M+1]= 310.1; DMSO-$d_6$ $^1$H NMR $\delta$ 2.89 (6H,s), 5.88 (1H,s), 6.26 (1H,s) 6.77 (2H,d), 7.65 (2H,d), 7.88 (1H,m), 8.41 (1H, s), 9.85 (1H, s); $^{13}$C NMR $\delta$ 38.0, 107.4, 111.6, 113.3, 114.5, 114.9, 125.5, 128.6, 129.3, 130.2, 133.3, 134.6, 136.8, 147.3, 155.8.

**Example 5.** 2-(4-(pyrrolidin-1-yl)phenyl)-7-methyoxyimidazo[2,1-*b*]benzothiazole (**5**).

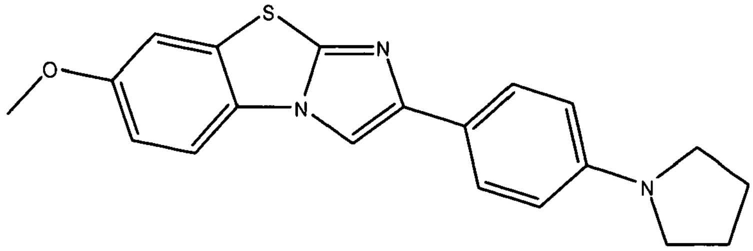

The title compound was prepared in a similar manner as given in Example 1 by using 1 mmol of 6-methoxybenzo[*d*]thiazol-2-amine and 2-bromo-1-(4-(pyrrolidin-1-yl)phenyl)ethanone to afford (295 mg, 84% yield in a purity >95% as measured by.HPLC). ESI-MS, [M+1]=350.2; DMSO-$d_6$ $^1$H NMR δ 1.97 (4H,t), 3.28 (4H,t), 3.85 (3H,s), 6.66 (2H,d), 7.28 (1H,d), 7.61 (2H,d), 7.83 (1H,s), 8.05 (1H,d), 8.78 (1H,s); $^{13}$C NMR δ 25.8, 48.4, 56.8, 108.3, 110.4, 112.6, 115.8, 126.4, 126.9, 127.5, 131.0, 131.5, 141.9, 145.9, 148.5, 158.6.

**Example 6.** 2-(4-(Pyrrolidin-1-yl)phenyl)-7-hydroxyimidazo[2,1-*b*]benzothiazole (**6**).

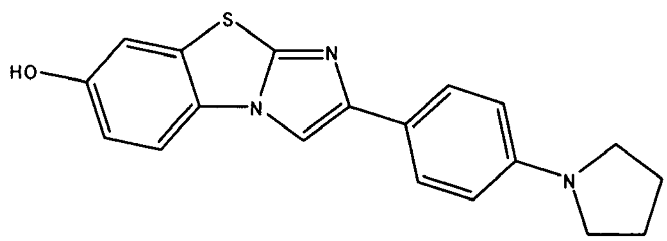

**Compound 5** 2-(4-(Pyrrolidin-1-yl)phenyl)-7-methyoxyimidazo[2,1-*b*]benzothiazole, (105mg, 0.3 mmol) was reacted with 1.5 molar equivalents of $BBr_3$ (1 molar solution) in 10 ml dichloromethane and microwave irradiation at 150˚C for 20 min. The reaction mixture was quenched by adding water and extracted with 2 × 10 ml $CH_2Cl_2$. The combined organic phase was washed with saturated Sodium bicarbonate solution (10 ml), dried over sodium sulphate and evaporated. The crude product, 2-(4-(pyrrolidin-1-yl)phenyl)-7-hydroxyimidazo[2,1-*b*]benzothiazole, was recrystallized from MeOH (89 mg, 88% yield) and further used for $^{11}$C-methylation reaction. ESI-MS, [M+1]= 336.1; DMSO-$d_6$ $^1$H NMR δ 1.86 (4H,s), 3.14 (4H,s), 5.32 (OH, br), 6.46 (2H,d), 7.12 (1H,d), 7.39 (2H,d), 7.61 (1H,s), 8.00 (1H,d), 8.84 (1H, s); $^{13}$C NMR δ 25.7, 48.4, 108.4, 111.9, 116.0, 116.7, 125.1, 127.0, 127.6, 131.4, 131.7, 140.1, 145.4, 148.0, 156.2.

**Example 7.** 4-(7-Methoxyimidazo[2,1-*b*]benzothiazol-2-yl)-benzenamine (**7**).

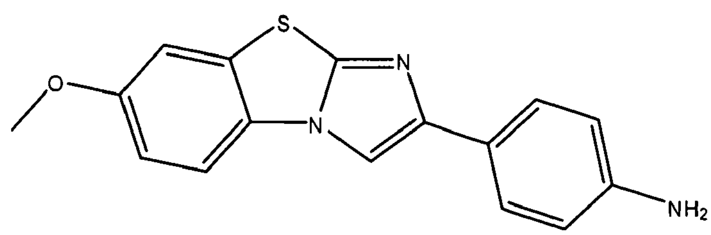

A mixture of 6-methoxybenzo[*d*]thiazol-2-amine (180 mg, 1 mmol) and 2-bromo-1-(4-nitrophenyl)ethanone (244 mg, 1 mmol) in 5 ml EtOH was heated for 1 h at reflux. The reaction mixture was cooled to room temperature, filtered, washed with 2 ml ether and the precipitate, 4-(7-methoxyimidazo[2,1-*b*]benzothiazol-2-yl)-nitrobenzene dried under vacuum (321 mg, 99% yield with HPLC purity of > 95%). Thereafter, a mixture of the nitro derivative (321 mg, 0.99 mmol) and $SnCl_2$ (5 eq., 690 mg) in 20 ml EtOH was heated for 2 h at reflux temperature under nitrogen. The reaction mixture was concentrated under vacuum and the residue taken to 200 ml ethyl acetate, the organic phase washed with 1 M NaOH solution, followed by water, and was subsequently dried over sodium sulphate and the solution concentrated. The crude product, 4-(7-methoxyimidazo[2,1-*b*]benzothiazol-2-yl)-benzenamine 7 was recrystallized from MeOH, dried under high vacuum and directly used as precursor for radiosynthesis of **[$^{11}$C]8.** LC-MS-ESI, [M+1] = 296.1

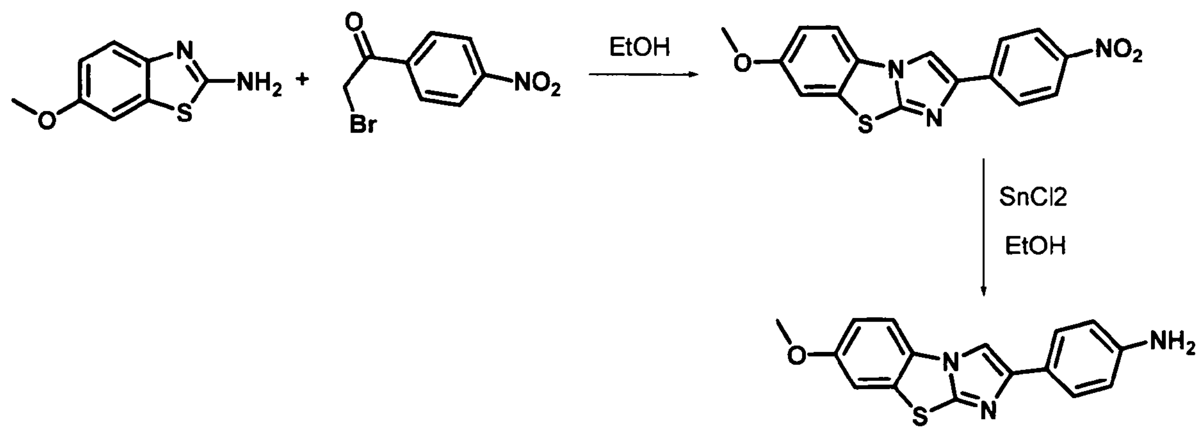


7

Synthesis of 7

**Example 8**. 2-(*p*-Methylaminophenyl)-7-methoxyimidazo[2,1-*b*] benzothiazole (**8**).

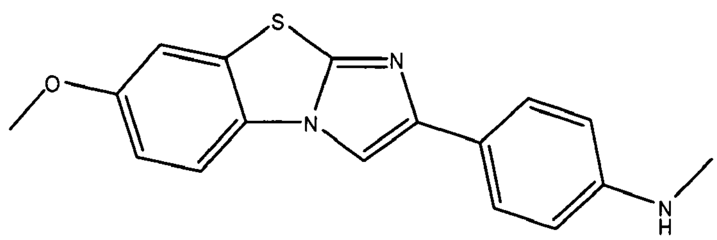

*Method 1:* Compound 7 (295 mg, 1 mmol) was dissolved in DMF (10 ml) and treated with potassium carbonate (anhydrous, 2 eq., 280 mg). One equivalent of methyl iodide was added to the mixture at 140 °C. The reaction mixture was cooled to room temperature, 50 ml water was added, and the resulting mixture was extracted with dichloromethane (3 × 50 ml). The combined organic phase was washed with brine, dried over sodium sulphate and concentrated. The crude product was purified by flash chromatography. ESI-MS, [M+1]= 310.1; $^{1}$H NMR (DMSO-$d_6$) δ 2.7 (d, J= 5 Hz, 3H), 3.3 (s, 3H), 5.7 (br s, 1H), 6.6 (d, J= 8.5 Hz, 2H), 7.1 (q, $J_A$= 8.5, $J_B$= 2.5 Hz, 1H), 7.6 (m,4H), 8.4 (s, 1H); $^{13}$C NMR (DMSO-$d_6$) δ 30.2, 56.3, 106.8, 109.9, 112.1, 114.1, 118.8, 122.2, 126.1, 126.6, 130.7, 146.1, 147.6, 149.7, 157.1.

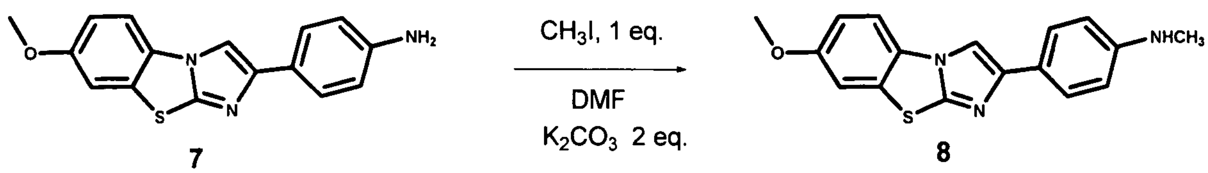


7

8

Synthesis of **8**

*Method 2:* Alternatively, **8** was prepared by reacting 6-methoxybenzo[*d*]thiazol-2-amine (180 mg, 1 mmol) with N-(4-(2-bromoacetyl)phenyl)acetamide (256 mg, 1 mmol) in 5 ml EtOH for 2h at reflux. The reaction mixture was cooled to room temperature, filtered, washed with 2 ml ether and the precipitate, *N*-acetyl-4-(7-methoxyimidazo[2,1-*b*]benzothiazol-2-yl)-benzenamine, **9** was dried under vacuum (318 mg, 94% yield, HPLC purity of >95%). **9** was methylated with 2 eq. methyltriflate in 30 ml acetone at 60°C in a for 10 min in a closed vial. After this, 200 ml water was added and

the solution filtered. The intermediate, **10,** was treated with 2M NaOH at 100˚C for 10 min. using microwave heating. Workup afforded **8** in > 95% purity (HPLC).

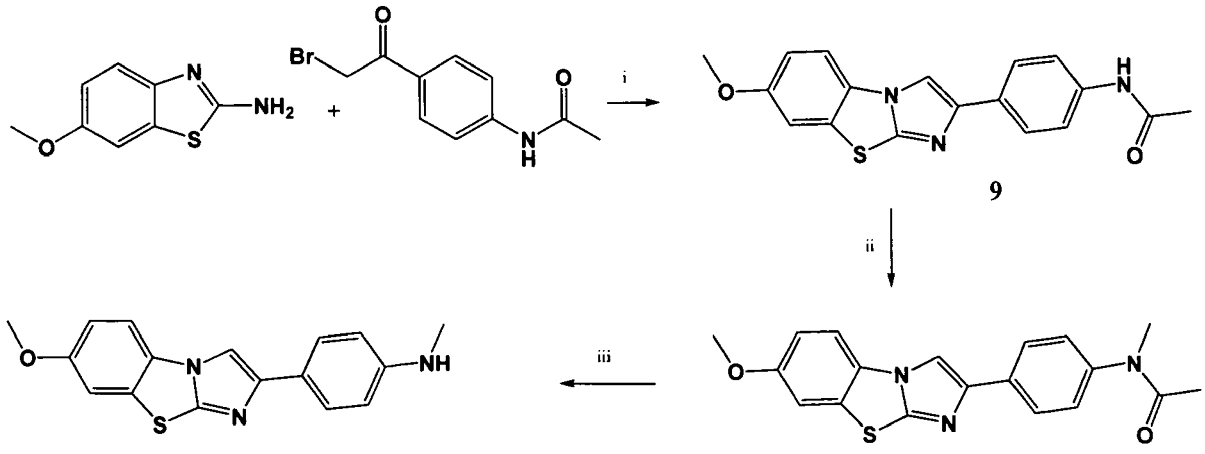


9

10

8

i EtOH reflux 2 h, ii Aceton, MeOTf, iii NaOH 2 M 100˚C.

**Example 9**. 2-(m-Fluoro-*p*-methoxyphenyl)-7-chloroimidazo[2,1-*b*] benzothiazole(**11**).

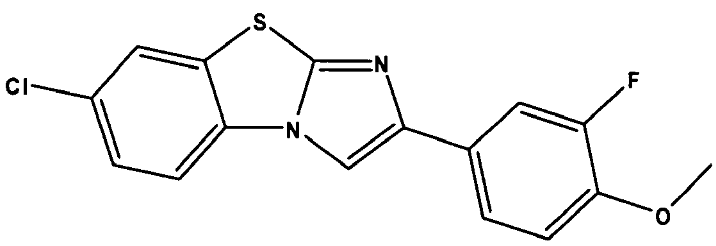

A mixture of 6-chlorobenzo[*d*]thiazol-2-amine (185 mg, 1 mmol) and 2-bromo-1-(3-fluoro-4-methoxyphenyl) ethanone (247 mg, 1 mmol) in 5 ml EtOH was heated overnight at reflux. The reaction mixture was cooled to room temperature, filtered, washed with 2 ml ether and the precipitate, 2-(*m*-fluoro-*p*-methoxyphenyl)-7-chloroimidazo[2,1-*b*] benzothiazole **11** was dried under vacuum (252 mg, 76% yield, purity of > 95%.as measured by HPLC). ESI-MS, [M+1] = 333.1; $^{1}$H NMR (DMSO-$d_6$) δ 3.88 (s, 3H), 7.24 (t, 1H), 7.63 (m, 3H), 7.95 (d, 1H), 8.22 (d, 1H), 8.72 (s, 1H); $^{13}$C NMR (DMSO-$d_6$) δ 57.0, 109.8, 113.0, 115.1, 121.8, 125.6, 127.7, 129.9, 131.6, 131.9, 146.1, 147.3, 147.4, 147.9, 151.2, 153.9.

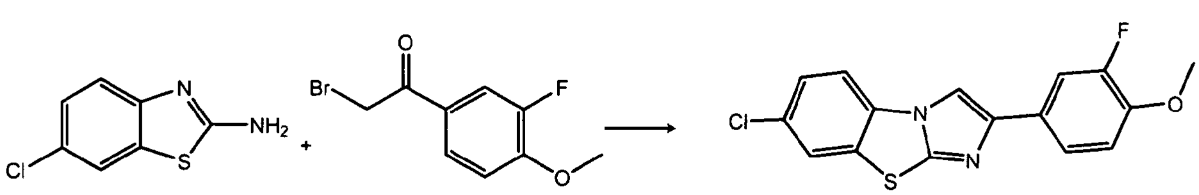

11

Preparation of **11**

**Example 10.** 2-(p-Methylaminophenyl)-7-(2-fluoroethoxy)imidazo[2,1-*b*]benzothiazole (**12**).

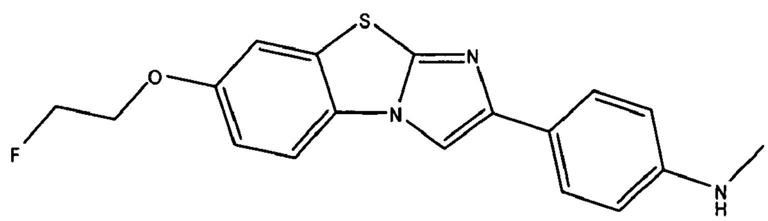

*N*-Acetyl-*N*-methyl-4-(7-methoxyimidazo[2,1-b]benzothiazol-2-yl)-benzenamine, **10** (105 mg, 0.3 mmol) was reacted with 2 equivalents of $BBr_3$ (1 molar solution) in 10 ml dichloromethane under microwave irradiation at 120°C for 30 min. The reaction mixture was quenched by adding water and thereafter extracted with 2 × 10 ml $CH_2Cl_2$. The combined organic phase was washed with saturated sodium bicarbonate solution (10 ml), dried over sodium sulphate and concentrated. The crude product, N-acetyl-N-methyl-4-(7-hydroxyimidazo[2,1-*b*]benzothiazol-2-yl)-benzenamine **13**, was deprotonated with NaH (2 eq.) in 10 ml DMF followed by reaction with 1 equivalent of 1-bromo-2-fluoroethane at 80°C. The reaction was followed by TLC and after 15 min no starting material was detected. The reaction mixture was cooled to room temperature. 100 ml water was added, and the mixture extracted with 3 × 50 ml ethyl acetate. The combined organic phase was washed with saturated sodium bicarbonate, brine, dried over sodium sulphate and concentrated. The product was dissolved in 5 ml EtOH and directly deprotected by treatment with 20 ml of a 2 M NaOH solution at 100°C for 10 min. using microwave heating. The reaction mixture was quenched with water and extracted with dichloromethane 3 × 50 ml, washed with saturated sodium bicarbonate, brine and dried over sodium sulphate. The organic phase was evaporated to give **15** (80 mg, 78% overall yield, purity > 95 %). ESI-MS, [M+1]= 342.1; $^1H$ NMR (DMSO-$d_6$) δ 2.71 (s, 3H), 4.32 (m, 2H, $J_{HF}$ = 32Hz), 4.79 (m, 2H, $J_{HF}$ = 48Hz), 5.75 (s, 1H), 6.59 (d, 12H), 7.18 (dd, 1H), 7.58 (d, 2H), 7.68 (d, 1H), 7.85 (d, 1H), 8.40 (s, 1H); $^{13}C$ NMR (DMSO-$d_6$) δ 30.6, 68.6, 83.8, 100.6, 107.2, 111.2, 112.6, 115.5, 115.1, 126.5, 129.6, 131.1, 139.9, 150.1, 154.3, 156.3.

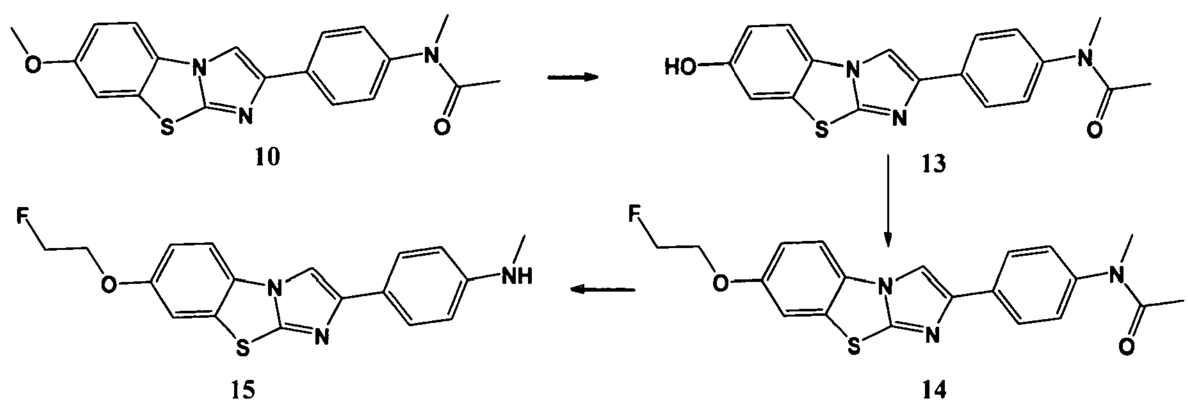

10

13

15

14

Synthesis of 15.

**Example 11. Compounds of the invention**

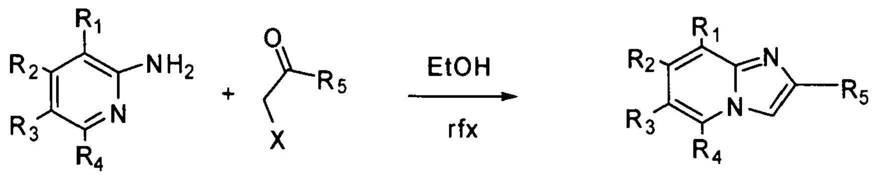


16 17 18

Syntheses of imidazo[2,1-*a*]pyridines.

Most of the new imidazo[2,1-*a*]pyridines **18** were synthesized through direct condensation of the appropriately substituted 2-aminopyridines **16** and the appropriately substituted α-bromoketones **17.** Treatment of **16** with 1.2 equivalents of **17** in ethanol at refluxing temperature afforded **18** in 25 to 93% yield.
Whereas in formula **16** $R_1$-$R_4$ is hydrogen, nitro, halide, hydroxyl, alkoxy, halo alkoxy 1', 2' or 3'amine, halo alkylamines, cyclic and acyclic groups and in formula **17,** $R_5$ is aryl, heteroaryl, bicyclic compounds and their substituted aromatic and heteroaromatic systems wherein substitutions are, in one or more positions, nitro, halide, hydroxyl, alkoxy, halo alkoxy 1', 2' or 3'amine, halo alkylamines, cyclic and acyclic groups.

**Example 12.** 6-chloro-2-(5-chlorothiophen-2-yl)imidazo[1,2-*a*]pyridine, (**19**).

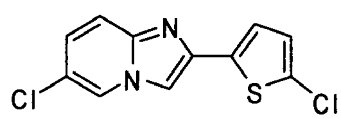

2-Amino-5-chloropyridine (128.5 mg, 1 mmol) and 2-bromo-1-(5-chlorothiophen-2-yl)ethanone (288 mg, 1.2 mmol) were stirred in reflux ethanol (5mL) for 4 h. The mixture was allowed to cool down to room temperature while stirring and then filtered. The white solid was washed with diethyl ether and dried under vacuum to give 218 mg of product. Yield 81%; > 99% HPLC purity; ESI-MS , [M+1]=269.0; DMSO-$d_6$ $^1$H NMR δ 8.88 (1H,s), 8.35 (1H,s), 7.67 (1H, d), 7.42 (1H,d), 7.32 (1H,d), 7.18 (1H, d); $^{13}$C NMR δ 143.2, 129.2, 128.9, 128.4, 1126.1, 125.3, 121.0, 117.4, 110.3.

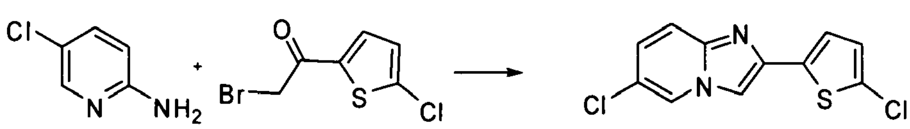

Syntheses of 19.

**Example 13** Compounds of the invention

The benzothiazoles **24** were prepared from 2-aminothiophenoles **21** and the appropriately substituted aldehydes **22** or acylchlorides **25** based on previously reported methods (Henriksen et al. J Med Chem 2007; 50: 1087-1089) afforded **24** in 46-92% yield. Similarly, the benzothiazoles **27** were prepared from 2-aminothiophenole **26** and the appropriately substituted aldehydes **22** or acylchlorides **25.**

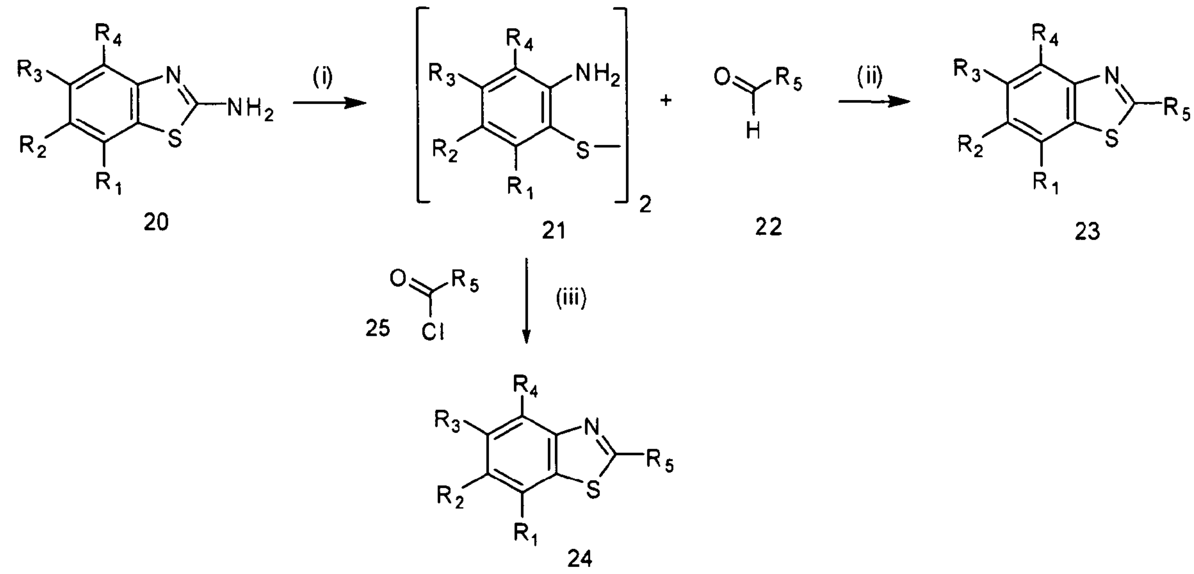


20 21 22 23

25

24

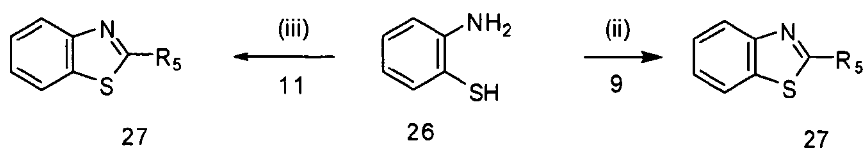


27 26 27

Reagents: (i)10N KOH, ethylene glycol 150° MW; (ii) DMSO, 170°, 20 min., (iii) NMP, 100°

Whereas in formula **20** and **21** $R_1$-$R_4$ is hydrogen, nitro, halide, hydroxyl, alkoxy, halo alkoxy 1', 2' or 3'amine, halo alkylamines, cyclic and acyclic groups and in formula **22** and **25,** $R_5$ is aryl, heteroaryl, bicyclic compounds and their substituted aromatic and heteroaromatic systems wherein substitution in one or more positions, is nitro, halide, hydroxyl, alkoxy, halo alkoxy 1', 2' or 3'amine, halo alkylamines, cyclic and acyclic groups.

**Example 14.** 2-(4-(6-chloropyridazin-3-yl)phenyl)-6-methoxybenzo[*d*]thiazole, **27a**

The 2-amino-5-methoxythiophenol (156 mg, 0.5 mmol) and 4-(6-chloropyridazin-3-yl)-benzaldehyde (218 mg, 1 mmol) were stirred at 170 ˚C in DMSO (3 mL) for 20 min. The reaction mixture was cooled to room temperature and poured into ice-water. The precipitate was filtered under reduced pressure, washed with diethyl ether and recrystallized from 70% ethanol to furnish 184.6 mg (52%) of **27a** as a pale-yellow solid with 98% HPLC purity; ESI-MS , [M+1]=354.1; DMSO-$d_6$ $^1H$ NMR δ 8.38 (2H,d), 8.13 (1H,d), 7.67 (2H,d), 7.42 (2H,m), 7.11 (1H,d), 7.07 (1H, d), 3.68 (3H, s).

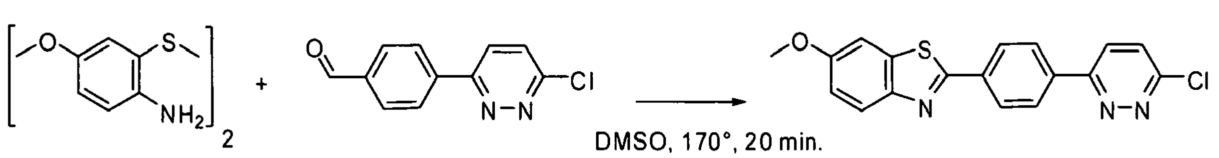


27a

Syntheses of 27a

**Example 15.** Determination of test compound's inhibition of *N*-[$^3$H-methyl]6-OH-BTA-1 binding to fibrils of A$\beta_{40}$ and A$\beta_{42}$.

Human A$\beta_{40}$ and A$\beta_{42}$ peptides (Bachem) were incubated at 0.5 mg/ml in a solution of 10 mM $Na_2HPO_4$, 1 mM EDTA (pH 7.4) at 37 ˚C for 48 h. The formation of fibrils was confirmed by microscopy and binding of [$^3$H]6-OH-BTA-1 ([$^3$H]PIB). Fibrils were either used immediately or aliquoted and subsequently stored at -80 ˚C until use.

Solutions of the test substances (> 95% purity according to analytical HPLC) or 6-OH-BTA-1 (> 95% purity according to analytical HPLC; ABX Biochemicals, Radeberg, Germany) and *N*-[$^3$H-methyl]6-OH-BTA-1 were prepared as 1-10 mM dimethyl sulfoxide (DMSO) stocks before dilution into assay buffer. The maximum final concentration of DMSO in the assays was 1%. All assays were performed in 10 mM $Na_2HPO_4$. The incubation was performed at 25 ˚C for 180 min. The bound and free fractions were separated by vacuum filtration through GF/B glass filters (Whatman, Maidstone, UK) using a Perkin Elmer harvester (PerkinElmer, 96 Micro B Filtermat) followed by 6 x 0.2 ml washes with ice cold phosphate buffer. Filters containing the bound ligand were counted with a liquid scintillation counter (Wallac Trilux, 1450 Microbeta).

The inhibition of [$^3$H]PIB binding to fibrils of $A\beta_{40}$ and $A\beta_{42}$ was determined at 100 nM concentration of the test compound in question.

**Example 16.** Determination of inhibition constants $K_i$ of test compounds

The inhibition of [$^3$H]PIB binding was measured for five different concentrations of the test compound in question, performed in duplicates. The determined $IC_{50}$ value was used for calculation of the inhibition constant, $K_i$, in the following manner

$$K_i = IC_{50}/(1 + ([L]/K_d))$$

were [L] is the concentration (4 nM) and $K_d$ (4.2 nM) of [$^3$H]PIB used in the assay

**Example 17.** *N*-$^{11}$C-methylation of primary and secondary amines

Cyclotron produced [$^{11}$C]$CO_2$ was converted to [$^{11}$C]$CH_3$I by the catalytic gas-phase iodination reaction via [$^{11}$C]$CH_4$ (GE Mel MicroLab). For further conversion to [$^{11}$C]$CH_3$OTf, the converted [$^{11}$C]$CH_3$I was distilled through a column of α-alumina impregnated with AgOTf.

10 μmol of the amine compound in question was dissolved in anhydrous acetone (250 μl). The vial was sealed was flushed with and maintained under argon. The [$^{11}$C]$CH_3$OTf produced, swept with a He-flow at 50 ml/min, was trapped in the reaction vial at room temperature. The reaction vial was warmed to 65 ˚C over 30 s and kept at this temperature for 3 min. Thereafter the reaction mixture was diluted with 1 ml of a mixture of MeCN:0.1 M ammonium formate (ratio in the range of 27.5:72.5 to 40:60, $^v/_v$), loaded into a 2 ml injection loop and transferred onto a Chromolith C18 1 (ID: 10 mm; length: 100 mm; Merck). The column was eluted with a mobile phase consisting of MeCN:0.1 M ammonium formate (ratio in the range of 27.5:72.5 to 40:60, $^v/_v$) at a flow rate of 10 ml / min. In-line HPLC detectors included a UV detector (Sykam) set at 254 nm and a radioactivity detector (Bioscan Flow-Count fitted with a PIN detector). For animal experiments, the fraction containing the product was collected and diluted 1:1 with water. The mixture was applied to a Sep-Pak C18 and the cartridge subsequently washed with 10 ml water. The product was eluted with ethanol and diluted to the desired concentration with phosphate buffered saline. The pH of the final solution was between 7 and 8. Radiochemical purities were > 95 % as determined by analytical HPLC.

**Example 18**. *O*-$^{11}$C-methylation of phenols

10 μmol of the phenolic compound was dissolved in anhydrous *N*'-*N*'-dimethyl-formamide (250 μl) containing 10 times molar excess of sodium hydride (NaH) and allowed to react for 5 min at ambient temperature. The solution was separated from the remaining NaH, transferred to a dry reaction vial. The vial was sealed, and subsequently flushed and maintained under argon. The [$^{11}$C]$CH_3$I produced, swept with a He-flow at 50 ml/min, was trapped in the reaction vial at ambient temperature. The reaction vial was warmed to 90 ˚C and kept at this temperature for 2-5 min. Thereafter the reaction mixture was diluted with 1 ml of MeCN:0.1 M ammonium formate (40:60, $^v/_v$), loaded into a 2 ml injection loop and transferred onto a Chromolith C18 1 (ID: 10 mm; length: 100 mm; Merck). The column was eluted with a mobile phase consisting of MeCN:0.1 M ammonium formate (40:60, $^v/_v$ ) at a flow rate of 10 ml / min. In-line HPLC detectors included a UV detector (Sykam) set at 254 nm and a γ-ray detector (Bioscan Flow-Count fitted with a PIN detector). For animal experiments, the fraction containing the product was collected and diluted 1:1 with water. The mixture was applied to a Sep-Pak C18 and the cartridge subsequently washed with 10 ml water. The product was eluted with ethanol and diluted to the desired concentration with phosphate buffered saline. The pH of the final solution was between 7 and 8. Radiochemical purities were > 95 % as determined by analytical HPLC.

**Example 19.** *O*-$^{18}$F-fluoroethylation of phenols.

[$^{18}$F]fluoride was produced through the $^{18}O(p,n)^{18}F$ nuclear reaction. The [$^{18}$F]fluoride was obtained in a 34 mM solution of $K_2CO_3$ (0.3 ml) and added to a 2 ml conical vial containing 0.5 ml dry MeCN and 15 mg (39.9 μmol) Kryptofix 2.2.2. The solvent was evaporated with heating under reduced pressure. Azeotropic drying was repeated three times with 0.5 ml portions of MeCN. 2-[$^{18}$F]fluoroethyltosylate was prepared by dissolving 5 mg (12 μmol) of ethylene glycol-1,2-ditosylate in 250 μl MeCN and adding the solution to the dried kryptate ($[K\subset 2.2.2]^+/^{18}F^-$), the vial was then sealed and heated at 90 °C for 5 min. 2-[$^{18}$F]fluoroethyltosylate was purified by reversed phase HPLC (Lichrosorb $C_{18}$ 5μ; 10 mm x 250 mm (CS-Chromatographie Service) eluted with MeOH / $H_2O$ (50:50, volume/volume, flowrate 4 ml/min; k' = 5.7; preparative radiochemical yield 55 ± 5 %). After on-line fixation of the product on a Strata X cartridge (33 μm; 30 mg / 1 ml; Phenomenex) and drying of the product by argon-flush, the product was eluted with 0.15 ml DMF into a reaction vial. 10 μmol of the phenolic compound in question was dissolved in anhydrous *N'*-*N'*-dimethylformamide (250 μl) containing 10 times molar excess of sodium hydride (NaH) and allowed to react for 5 min at ambient temperature. The solution was separated from the remaining NaH, transferred to a dry reaction vial. The vial was sealed was flushed with and maintained under argon. 2-[$^{18}$F]fluoroethyl-tosylate was led into the reaction vial at room temperature. The reaction vial was warmed to 90 °C and kept at this temperature for 5 min. Thereafter the reaction mixture was diluted with 1 ml of MeCN:0.1 M ammonium formate (40:60, $^v/_v$ ), loaded into a 2 ml injection loop and transferred onto a Chromolith C18 (ID: 10 mm; length: 100 mm; Merck). The column was eluted with a mobile phase consisting of MeCN: 0.1 M ammonium formate (40:60, $^v/_v$) at a flow rate of 10 ml / min. In-line HPLC detectors included a UV detector (Sykam) set at 254 nm and a radioactivity detector (Bioscan Flow-Count fitted with a PIN detector). For animal experiments, the fraction containing the product was collected and diluted 1:1 with water. The mixture was applied to a Sep-Pak C18 and the cartridge subsequently washed with 10 ml water. The product was eluted with ethanol and diluted to the desired concentration with phosphate buffered saline. The pH of the final solution was between 7 and 8. Radiochemical and chemical purities were > 95 % as determined by analytical HPLC.

**Example 20.** Preparation of $^{18}$F-fluorinated compounds by direct fluorination

[$^{18}$F]Fluoride was obtained in a 34 mM solution of $K_2CO_3$ (0.3 ml) and added to a 2 ml conical vial containing 0.5 ml dry MeCN and 15 mg (39.9 μmol) Kryptofix 2.2.2. The solvent was evaporated with heating under reduced pressure. Azeotropic drying was repeated three times with 0.5 ml portions of MeCN. 10 μmol of the precursor in question was dissolved in 300 μl MeCN and adding the solution to the dried kryptate ($[K\subset 2.2.2]^+/^{18}F^-$), the vial was then sealed and heated at 90 °C for 5 min.

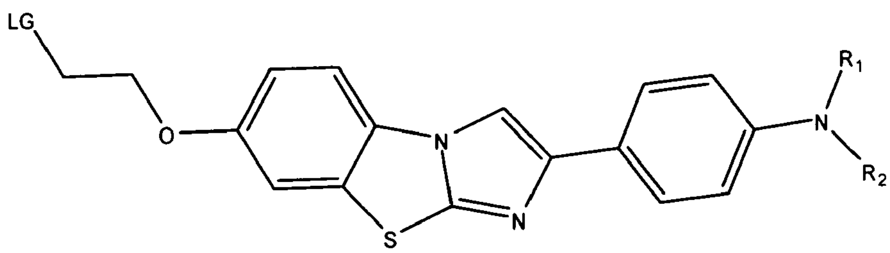

| LG | $R_1$ | $R_2$ | Temperature (°C) | Reaction time (min) | Yield[a] % |
|---|---|---|---|---|---|
| I | $CH_3$ | H | 90 | 5 | 32 |
| Br | $CH_3$ | H | 90 | 5 | 49 |
| OTs | $CH_3$ | H | 90 | 5 | 61 |
| Br | $CH_3$ | $CH_3$ | 90 | 5 | 48 |
| OTs | $CH_3$ | $CH_3$ | 90 | 5 | 58 |

[a] Analytical yield.

**Example 21**. Preparation of $^{18}F$-fluorinated compounds by direct fluorination

[$^{18}F$]Fluoride was obtained in a 34 mM solution of $K_2CO_3$ (0.3 ml) and added to a 2 ml conical vial containing 0.5 ml dry MeCN and 15 mg (39.9 μmol) Kryptofix 2.2.2. The solvent was evaporated with heating under reduced pressure. Azeotropic drying was repeated three times with 0.5 ml portions of MeCN. 10 μmol of the precursor in question was dissolved in 300 μl MeCN and adding the solution to the dried kryptate ([Kc⊂2.2.2]$^+$/$^{18}F^-$), the vial was then sealed and heated at a desired combination of temperature and reaction time. T

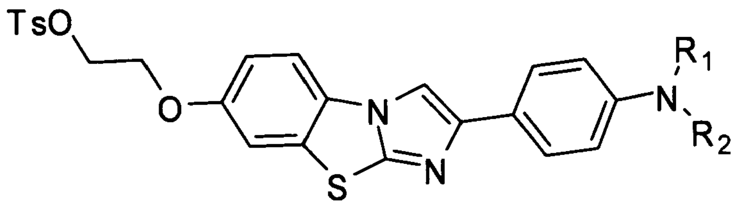

| $R_1$ | $R_2$ | Temperature (˚C) | Reaction time (min) | Yield[a] % |
|---|---|---|---|---|
| $CH_3$ | H | 80 | 5 | 32 |
| $CH_3$ | H | 90 | 2 | 28 |
| $CH_3$ | H | 90 | 5 | 49 |
| $CH_3$ | H | 90 | 10 | 57 |
| $CH_3$ | H | 90 | 15 | 54 |
| $CH_3$ | H | 100 | 2 | 34 |
| $CH_3$ | H | 100 | 5 | 52 |
| $CH_3$ | H | 100 | 10 | 48 |

[a] Analytical yield.

**Example 22.** Preparation of $^{18}F$-fluorinated compounds by direct fluorination

[$^{18}F$]Fluoride was obtained in a 34 mM solution of $K_2CO_3$ (0.3 ml) and added to a 2 ml conical vial containing 0.5 ml dry MeCN and 15 mg (39.9 μmol) Kryptofix 2.2.2. The solvent was evaporated with heating under reduced pressure. Azeotropic drying was repeated three times with 0.5 ml portions of MeCN. 10 μmol of the tosyl-precursor in question was dissolved in the solvent of choice and added the solution to the dried kryptate ([K⊂2.2.2]$^+$/$^{18}F^-$). Finally, the vial was then sealed and heated at 90 ˚C for 5 min. T

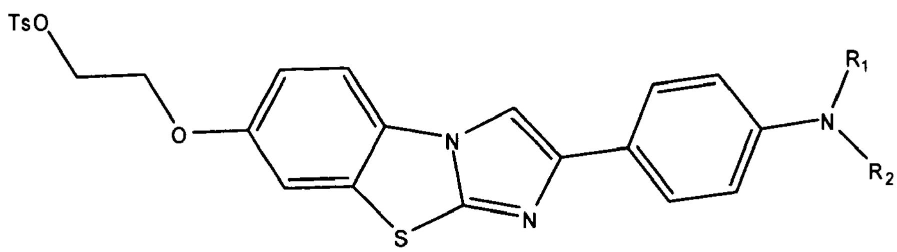

| $R_1$ | $R_2$ | Temperature (˚C) | Solvent | Yield[a] % |
|---|---|---|---|---|
| $CH_3$ | H | 90 | DMSO | 23 |
| $CH_3$ | H | 90 | DMF | 36 |
| $CH_3$ | H | 90 | DMAA | 42 |

(continued)

| $R_1$ | $R_2$ | Temperature (˚C) | Solvent | Yield[a] % |
|---|---|---|---|---|
| $CH_3$ | H | 90 | MeCN | 57 |

[a] Analytical yield.

**Example 23. Preparative $^{18}$F-radiosynthesis.**

[$^{18}$F]Fluoride was obtained in a 34 mM solution of $K_2CO_3$ (0.3 ml) and added to a 2 ml conical vial containing 0.5 ml dry MeCN and 15 mg (39.9 μmol) Kryptofix 2.2.2. The solvent was evaporated with heating under reduced pressure. Azeotropic drying was repeated three times with 0.5 ml portions of MeCN. 10 μmol of the tosyl-precursor in question was dissolved in 300 μl MeCN and adding the solution to the dried kryptate ([K⊂2.2.2]$^+$/$^{18}F^-$), the vial was then sealed and heated at 90 ˚C for 5 min. Thereafter the reaction mixture was diluted with 1 ml of a mixture of MeCN:0.1 M ammonium formate (ratio in the range of 27.5:72.5 to 40:60, $^v/_v$ ), loaded into a 2 ml injection loop and transferred onto a Chromolith C18 1 (ID: 10 mm; length: 100 mm; Merck). The column was eluted with a mobile phase consisting of MeCN:0.1 M ammonium formate (ratio in the range of 27.5:72.5 to 40:60, $^v/_v$ ) at a flow rate of 10 ml / min. In-line HPLC detectors included a UV detector (Sykam) set at 254 nm and a radioactivity detector (Bioscan Flow-Count fitted with a PIN detector). For animal experiments, the fraction containing the product was collected and diluted 1:1 with water. The mixture was applied to a Sep-Pak C18 and the cartridge subsequently washed with 10 ml water. The product was eluted with ethanol and diluted to the desired concentration with phosphate buffered saline. The pH of the final solution was between 7 and 8. Radiochemical purities were > 95 % as determined by analytical HPLC.

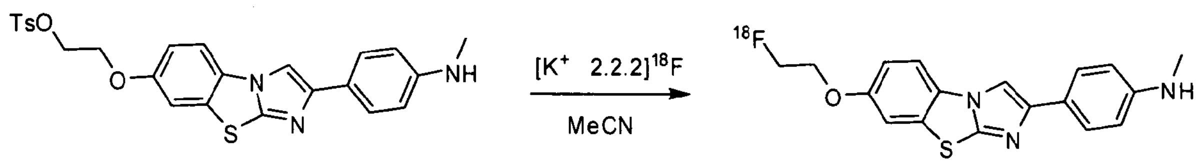


$^{18}$F-**15**

A typical applied amount of $^{18}$F-labeled tracer for the relevant studies is 2-3 mCi (74-111 MBq). This is based on the experience with a broad range of existing compounds for imaging of structures in the human central nervous system (CNS), including the four compounds that have been evaluated in humans for imaging of amyloid plaque in patients with Alzheimer's disease. The $^{18}$F-labeled compounds in question are prepared in a specific activity of > 3000 mCi/μmol (111 GBq/μmol). Thus, an expected injected amount of the substances is < 1 × $10^{-9}$ mol.

**Example 24. Measurement of log*P***

Apparent drug lipophilicity was determined by a conventional partition method between 1-octanol and phosphate buffered saline (PBS), pH 7.4. The 1-octanol was saturated with PBS before use. Briefly, the no-carrier-added "C- or $^{18}$F-labeled compound in question, contained in 4 ml PBS, was added 4 ml of 1-octanol in a 20 ml test tube. The tube was sealed and vigorously shaken at room temperature for 2 min. The mixture was then centrifuged at 3000 *g* for 10 min. A 20 μl aliquot from each of the two phases were drawn and their radioactivity content was determined in a NaI(TI) well-type detector. The log $P_{oct/PBS}$ was calculated as follows:

Log$P_{oct/log}$ (radioactivity concentration in the 1-octanol phase/radioactivity concentration in the PBS phase). The reported values represent the mean of three independent measurements.

**Example 25**. Biodistribution studies in mice

The experiments were carried out with the approval of the institutional animal care committee (Regierung von Oberbayern, Munich, Germany) and in accordance with the German Animal Welfare Act (Deutsches Tierschutzgesetz). Animal husbandry followed the regulations of European Union (EU) guideline No. 86/609.

Biodistribution studies were performed in male Balb-C mice (body weight of 19-25 g). The mice were injected in a lateral tail vein with 10-12 MBq of a high specific activity (> 37 GBq /μmol) $^{11}$C-labeled compound contained in 0.1-0.15 ml of a solution of isotonic phosphate buffered saline. Groups of mice were sacrificed at 5-30 min post-injection.

The radioactivity of weighed tissue samples was measured in a γ-counter. Data are expressed as percent of the injected dose per gram tissue (% I.D./g; mean ± sd, n = 4-5).

**Example 26.** Speciation of radioactive compounds in tissue of mice

Male Balb-C mice were injected with 21-32 MBq of the compound of interest. At 10 and 30 min after injection the animals were sacrificed and the tissue of interest was dissected. The procedure used for the analysis of brain radioactivity was as follows: Brain tissue homogenates were prepared immediately after dissection by mechanical homogenization of nitrogen-frozen samples followed by addition of 1 ml of phosphate buffered saline. The mixture was vigorously vortexed, and 1 ml of MeCN was added. After centrifugation for 5 min at 6.000•*g*, the supernatant was collected. Approximately 0.1 ml of the supernatant solution was analyzed using radio-HPLC [Nucleosphere 100, 5 μm; 10 x 150 mm (CS-Chromatographie); MeCN / 0.1 M ammonium formate 60:40, $^{v}/_{v}$]. The outlet of the column was connected in-line with a solid-phase scintillation counter.

The amount of intact tracer ($T_i$) was calculated as follows:

$$T_i = [F_T / F_T + F_M] \times E_E \times E_R \times 1 \cdot 10^{-2}$$

where $F_T$ [%] represents the amount of intact tracer and $F_M$ [%] the amount of metabolites as determined by radio-HPLC, corrected for extraction efficiency $E_E$ [%] from the plasma samples and the recovery $E_R$ [%] of activity from the HPLC. The extraction efficiency was in the range 68-92 % among the compounds investigated.

**Example 27.** Investigation of in vivo regional brain uptake of mice by means of small animal PET.

APP/PS1 transgenic mice (B6;CB-Tg(Thy1-PSEN1*M146V/Thy1-APP*swe)-10Arte) (Artemis Pharmaceuticals, Cologne, Germany) on a congenic C57BL/6J genetic background were used in the experiments. Anesthesia was begun 15 min ahead of experimental procedures by placing the animal in a cage ventilated with isoflurane (3 %) and oxygen (3.5 l/min) with a pre-calibrated vaporizer. Anesthesia was maintained by 0.6 % to 2 % isoflurane and 3.5 l/min oxygen via a nose cone, such that the respiratory rate stayed at 80-100 per min. Body temperature was held at 37 ˚C with a temperature-controlled heating pad.

The transgenic animals were compared with commercially available age- and gender-matched controls (female mice by Harlan-Winkelmann, Borchen, Germany and male mice by Janvier, Le Genest-St-Isle, France), females weighing 26.1±2.0g and males weighing 32.2±2.5g.

All injections were performed under isoflurane inhalation anesthesia. An application catheter system for reliable intravenous access to the lateral tail veins was set up using 30-gauge needles, polythene tubing with 0.28 mm inner diameter, superglue and 1 ml syringes. To achieve long-term access an elastic hollow vessel-loop was used as a tourniquet. The catheter and syringe were initially filled with isotonic sodium chloride solution. The functional catheter was stabilized at the injection site with superglue.

All small-animal PET data were acquired with a microPET FOCUS F120 scanner (Siemens Medical Solutions, Malvern, USA) (Tai et al., 2005).

The tracer in question was applied in a volume of 124±73μl injected via the catheter system intravenously in a slow bolus, followed by flushing with saline solution (84±26μl isotonic sodium chloride solution 0.9 %). Total applied volume was 253±76μl. This approach provided that registered prompts at the end of application ranged between 150000 to 200000, ensuring a dead time <5 % at 30min p.i.. Activities in the syringe were measured immediately before and after injection with a Capintech dose calibrator. Dynamic data acquisition was performed in 3D listmode starting immediately with injection of the tracer.

The emission data were normalized and corrected for the effects of scatter and attenuation. The resulting sinogram was reconstructed with FBP (filtered back-projection using a ramp filter with a cut-off at the Nyquist frequency) into 17 frames (5 x1 min, 5 x 2 min, 5 x 5 min, 2 x 10min). The image volume consisted of 128 x 128 x 95 voxels, with a size of 0.433 x 0.433 x 0.796 $mm^3$ per voxel.

Example 28. Preparation of mouse CNS for ex vivo evaluation

Brain tissue was rapidly frozen in fine-crushed dry ice and stored air-tight at -75 C. For tissue analyses, every whole and half brain was mounted on a freezing medium and cut on a cryostat. The frozen sections were mounted on dilute poly-L-lysine hydrobromide coated (mol wt > 300.000, (1:50) 0.01 % w/v in water) microscopy slides. After drying at ambient conditions the remaining slides were stored at -75 ˚C until assayed.

**Example 29.** Thioflavin-T staining

Deep frozen mouse brain sections were dried in ambient air for 15 min, immersion-fixed in 4 % ($^w/_v$) paraformaldehyde (PFA) in phosphate buffered saline (PBS, pH=7.4) for 20 min at RT (20-22 °C). Sections were equilibrated in water twice for 2 min.

Thioflavin T (ThT) was dissolved at 1 % ($^w/_v$) in water, and the solution was filtered. Sections were immersed in 1 % ThT for 30 min at RT and kept dark, rinsed for 3 min in water, and differentiated in two changes of 80 % ethanol (5 min and 1 min), washed again in three changes of water (2 min each) and mounted in ProLong Gold antifade mounting medium (Invitrogen, Karlsruhe, Germany) with coverslips.

**Example 30.** Immunofluorescence for $A\beta_{40}$ and $A\beta_{42}$

Immunofluorescence stainings of amyloid plaques were carried out selectively for $A\beta_{40}$ and $A\beta_{42}$ components. For stainings, sections were prepared with 70 % formic acid for 20 min and washed four times. Then, they were blocked with 3 % bovine serum albumin in PBS for 15 min and then probed with the two primary antibodies (G2-13 (The Genetics, Schlieren, Switzerland)) and AB2 (Araclon Biotech, Zaragoza, Spain)) diluted in 1 % BSA/PBS over night at 4 °C, washed with PBS, blocked again with 3 % BSA/PBS and incubated with two fluorophore-conjugated secondary antibodies (A488-D-Rb (Invitrogen, Karlsruhe, Germany) and Cy5-D-Ms (Jackson ImmunoResearch, Suffolk, Great Britain). After a short wash, nuclei were stained by adding 0.5 μM DAPI for 3 min. After the final washing steps the tissue was coverslip-mounted with ProLong Gold Antifade mounting medium.

**Example 31.** Ex vivo analysis of regional distribution in brain by autoradiography

Digital autoradiographic images with a field of view of 24x 32 mm were taken with the M40 series of μ-ImagerTM (Biospace lab, Paris, France) using 10 x 10 cm scintillating foils with 13 ± 1.5 μm thickness (Applied Scintillation T Administration of [$^3$H]PIB and radiolabeled test compounds for autoradiography

For *ex vivo* autoradiographical assessment the radiolabeled tracer was coinjected with [$^3$H]PIB (specific activity: 2.78 TBq/mmol, radiochemical purity > 97 %) into live animals. The animals were killed and the full brain was removed within 8 ± 2 min post-mortem, rapidly frozen in fine-crushed dry ice prior to sectioning.

echnologies, Harlow, England). The resolution with tritium is 20 μm, the detection threshold for tritium is 0.4 cpm/mm$^2$ and the smallest pixel size is 1 μm.

Instrument acquisition was controlled with μ-Acquisition software. The processing, quantitation and data management was handled with β-Vision + software (both by Biospace lab).

Deep frozen CNS and whole head sections were dried in ambient air for 30 min and covered with the scintillation foil. Three full axial sections were acquired in one scan. An optical image was taken first. A total of 3 million counts were obtained for each sample.

**Example 32.** Fluorescence microscopy

Fluorescence microscopy was performed with an AxioImager Z.1 microscope (Carl Zeiss, Göttingen, Germany). Entire-view micrographs of axial whole mouse brain sections were recorded through a 10*/0.3 EC Plan Neofluar Zeiss lens and Zeiss filter sets no.49 (DAPI), no. 38 (HE Green Fluorescent Protein), no.43 (HE DsRed), no. 47 (HE Cyan Fluorescent Protein) and no. 50 (Cy5). For the entire view, MosaiX pictures were created with a Zeiss CAN-Bus motor stage (Merzhäuser, Germany) and digitalized with an AxioCam MRm Rev. 3.0 (Carl Zeiss). AxioVision software, release 4.6, was used for acquisition and processing. The single micrographs were stitched to full-section view with the AxioVision MosaiX module.

**Example 33.** Processing of μPET data

All raw PET data sets were loaded into Amide 0.8.22 software. Each PET data set was cropped to contain the head and neck only. PET original data and scale formats (32-bit float with per plane scale factor and 16-bit unsigned short with single scale factor, respectively) were preserved for the cropped output. The data sets were worked on in tri-linear interpolation type.

**Example 34.** Inhibition of [$^3$H]binding to AD post-mortem brain tissue

AD post-mortem brain tissue was obtained from the Brain Bank Germany. The neuropathological diagnosis was confirmed by current criteria (Braak stage VI). The presence and localization of plaques on the sections was

confirmed with immunohistochemical staining with fluorescent monoclonal anti-Aβ antibodies: A4 (DAB) and (Apaap). The frozen sections were incubated with [$^{3}$H]PIB (1-2 $\times$ 10 $^{6}$ cpm/ml) for 1 h at room temperature with and without a test compound present at 100 nM concentration.

## Claims

1. A tracer for aggregates of amyloid peptides comprising the structure

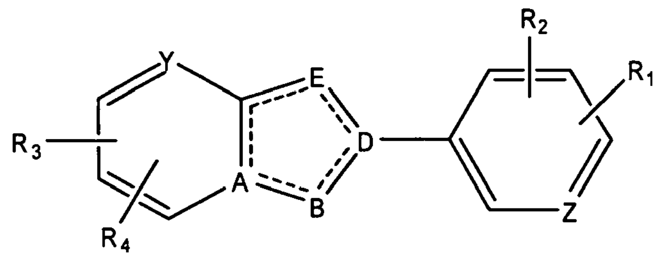

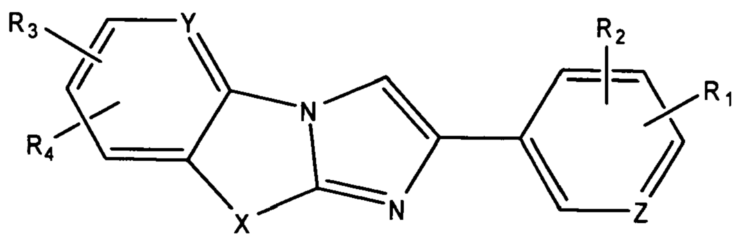

in which $R_1$, $R_2$, $R_3$ and $R_4$ are the same or different and can independently be H, F, Cl, Br, I, CN, $CF_3$, alkyl, alkynyl, alkoxy, monoalkylamine, dialkylamine, hydroxyalkyl, haloalkyl, alkylthio, alkylsulfonyl, aryl, heterocycles, heteroaryl, carboxy, esterified carboxy, $OR_6$, $NR_5$,$R_6$ or $R_5$,$R_6$ can be $C_nH_{2n}$+1 or -$CH_2$-CH=CH-halo in which halo can be any halogen and $R_6$ can be $C_nH_{2n}$+1, -[$CH_2$-$CH_2$-O]$_m$-$R_5$ where n and m can independently be an integer in the interval 0-7, A and D can independently be N or C. E, Y and Z can independently be CH or N; B can be S, O N or CH; X can be N, S, or O, and the tracers of invention can bind to and detect aggregates of amyloid peptides; preferably, the alkyl is a $C_1$ to $C_{10}$ alkyl, more preferably a $C_1$ to $C_8$ alkyl and even more preferably a $C_1$ to $C_6$ alkyl and this carbon chain may optionally be substituted or interrupted by heteroatoms like N, O and S with interruptions by up to four heteroatoms within the carbon chain being preferred, including ethers, thioethers, amines and polyethylene glycols (PEGs), aliphatic chains with heteroatom interruptions include PEG with 2 to 6 PEG groups and their corresponding polythioether equivalents being preferred or the PEG substituents may contain a halogen substitution, preferably a terminal halogen substitution.

2. A tracer for aggregates of amyloid peptides of claim 1, wherein the compound comprises the formula:

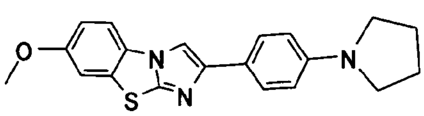

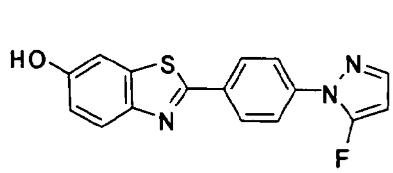

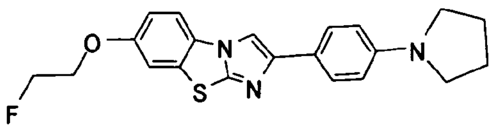

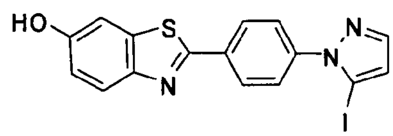

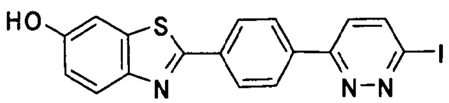

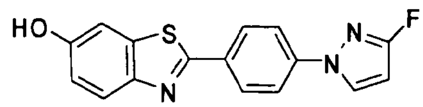

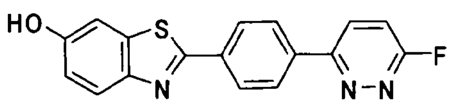

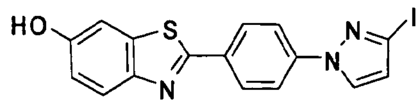

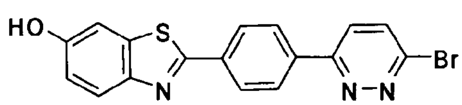

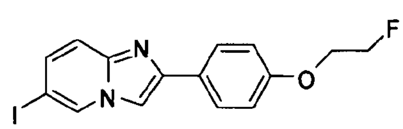

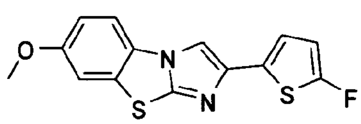

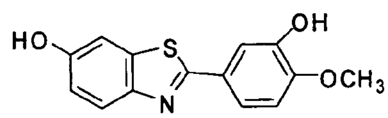

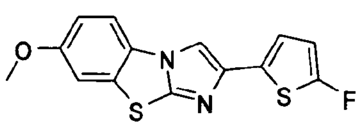

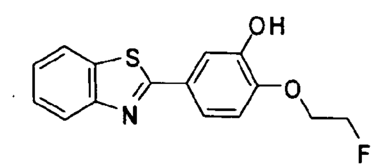

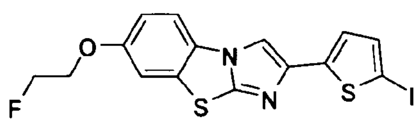

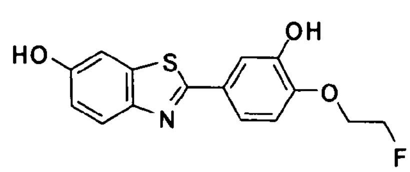

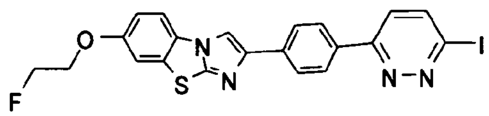

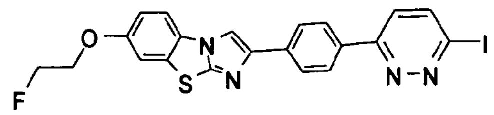

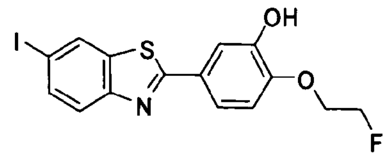

**3.** A method for the preparation of a tracer of claim 1 comprising the general formula II

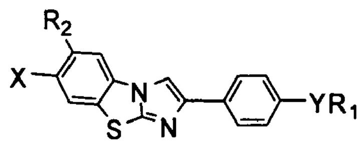

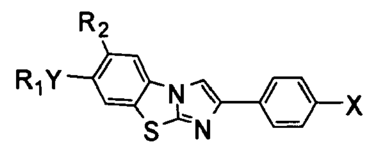

wherein

X = F, Br, I
Y = O, N, S
$R_1$ =H, alkyl, alkenyl, haloalkyl, hydroxyalkyl, polymers of ethylene glycol
$R_2$ = H, alkyl, alkenyl, haloalkyl, hydroxyalkyl

by fluorinating a derivative of the general formula I

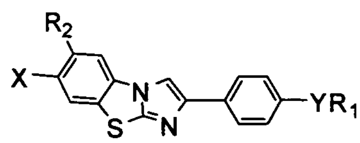

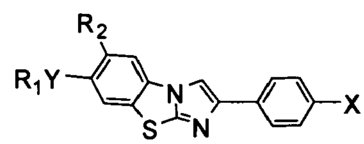

wherein

X = F, Br, I
Y = 0, N, S
$R_1$ =H, alkyl, alkenyl, haloalkyl, hydroxyalkyl, polymers of ethylene glycol each containing a leaving group.
$R_2$ = H, alkyl, alkenyl, haloalkyl, hydroxyalkyl each containing a leaving group.

with [$^{18}$F]fluoride.

**4.** The method according to claim 3, wherein the leaving group is selected from the group consisting of halogen atoms (chlorine, bromine, iodine), tosylsulfonate, trifluoromethanesulfonate, tolylsulfonatetriflate, tosylate, trialkyl ammonium, nitro and azide.

**5.** The method according to any of the preceding claim 3 or 4, wherein the fluorination is performed in a solvent, preferably an aprotic dipolar solvent.

**6.** The method according to claim 5, wherein the solvent is selected from the group consisting of dimethylformamide (DMF), tetrahydrofurane, (THF), dimethylsulfoxide (DMSO), acetonitrile and acetamide.

**7.** The method according to any of the preceding claims 3 to 6, wherein the fluorination is performed under microwave

heating.

**8.** The method according to any of the preceding claims 3 to 7, wherein the fluorination is performed at a temperature in the range of .25-200˚C, preferably 25-175 ˚C, more preferably 50-150 ˚C.

**9.** The method according to any of the preceding claims 3 to 8, wherein the ratio of $[^{18}F]$fluoride to the precursor derivative is in the range of 1:1,000-100,000, preferably in the range of 1:1,000-10,000 and more preferably in the range of 1:2,000-5,000.

**10.** The method according to any of the preceding claims 3 to 9, wherein a compound of one of the general formulas is comprising the following formulas:

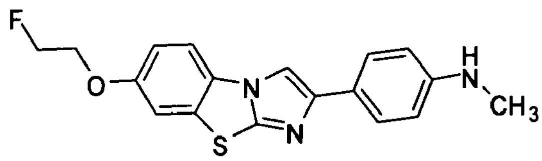

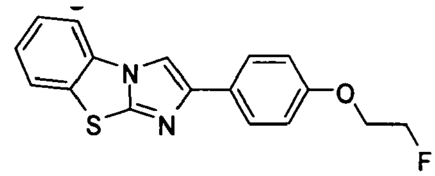

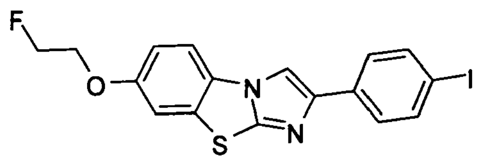

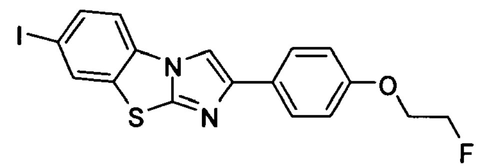

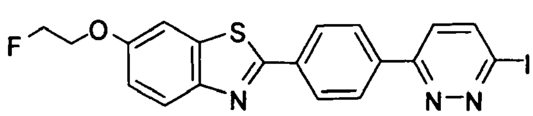

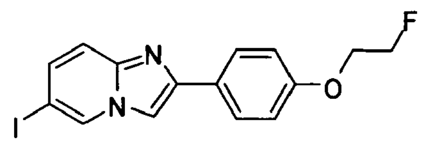

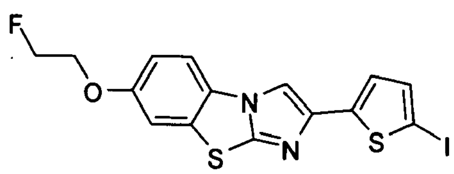

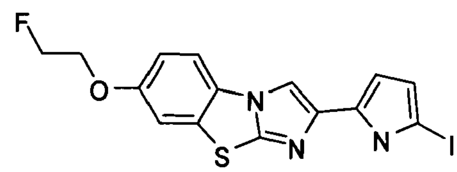

**11.** A tracer for aggregates of amyloid peptides of claim 1 wherein at least one of $R_1$, $R_2$, $R_3$ and $R_4$ independently comprises $^{11}C$, $^{18}F$, $^{19}F$, $^{75}Br$, $^{76}Br$, $^{123}I$, $^{131}I$, $^{124}I$.

**12.** Use of a tracer according to the present invention for the preparation of a diagnostic compound for the imaging and quantification of amyloidosis in Alzheimer's disease, type II diabetes, Down's syndrome, Creutzfeldt-Jacob disease, prion mediated diseases, amyloid polyneuropathy and amyloid cardiomyopathy.

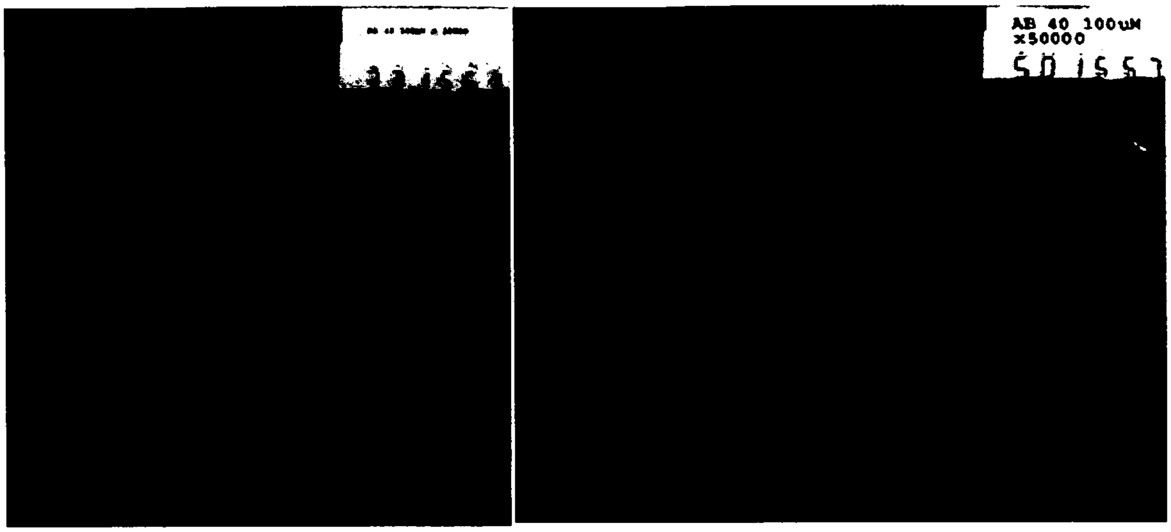


A

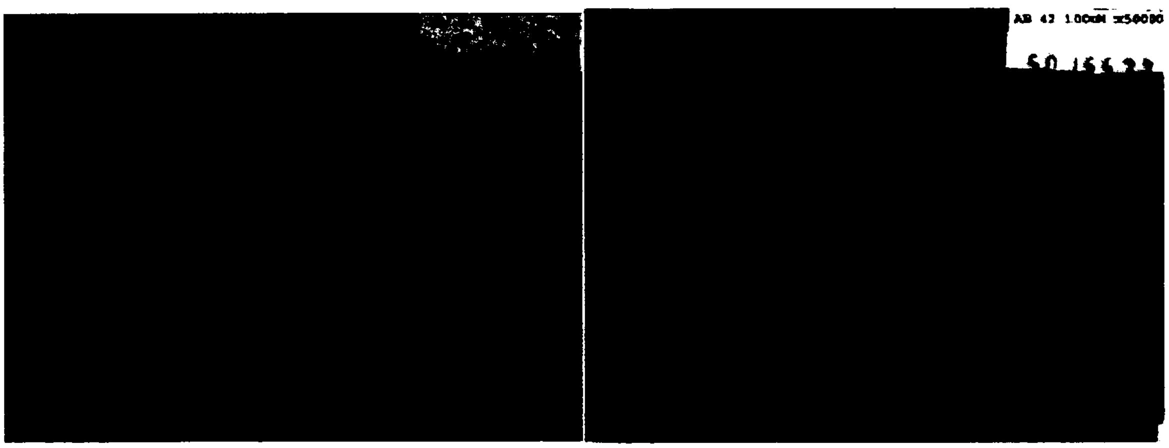


C

Fig. 1.

Fig 2.

| Structure | $A\beta_{40}$ inhibition% | $A\beta_{42}$ inhibition% |
|---|---|---|
| 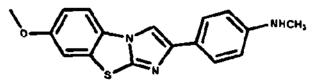 | 95 | 97 |
| 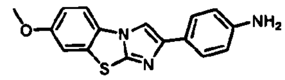 | 93 | 90 |
| 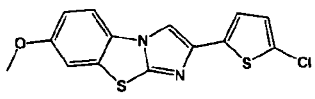 | 85 | 93 |
| 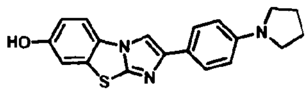 | 91 | 91 |
| 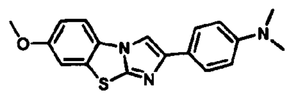 | 89 | 91 |
| 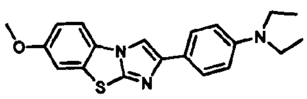 | 79 | 91 |
| 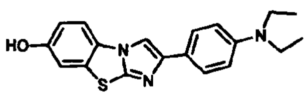 | 79 | 91 |
| 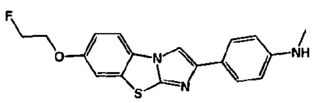 | 83 | 90 |
| 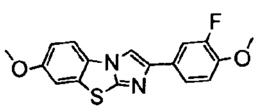 | 82 | 90 |
| 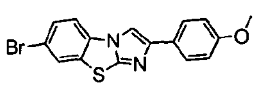 | 92 | 86 |
| 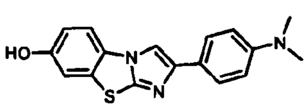 | 85 | 86 |
| 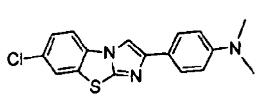 | 86 | 85 |
| 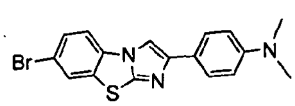 | 87 | 83 |
| 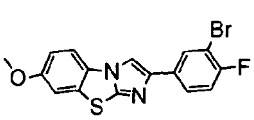 | 50 | 82 |

| | | |
|---|---|---|
| 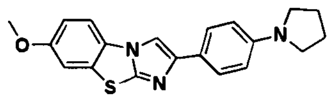 | 91 | 82 |
| 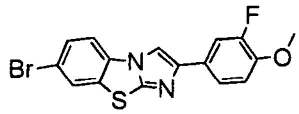 | 63 | 82 |
| 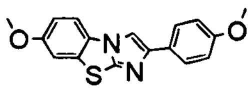 | 77 | 81 |
| 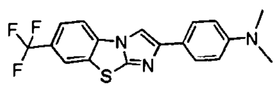 | 74 | 80 |
| 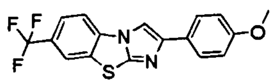 | 55 | 79 |
| 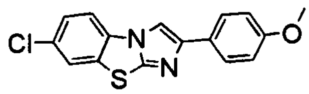 | 45 | 78 |
| 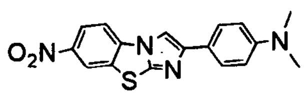 | 78 | 77 |
| 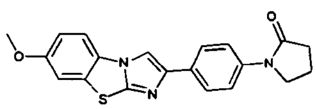 | 56 | 77 |
| 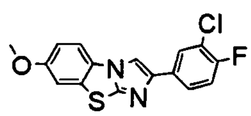 | 57 | 75 |
| 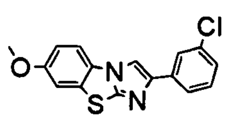 | 50 | 75 |
| 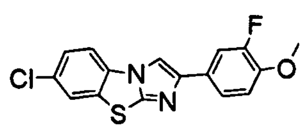 | 41 | 74 |
| 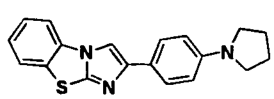 | 76 | 74 |
| 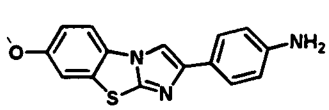 | 57 | 74 |

| | | |
|---|---|---|
| 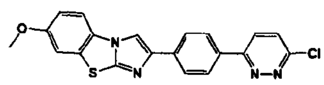 | 80 | 73 |
| 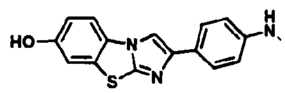 | 48 | 73 |
| 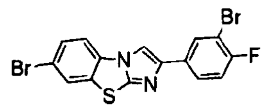 | 43 | 70 |
| 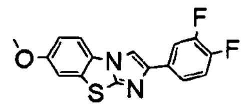 | 48 | 68 |
| 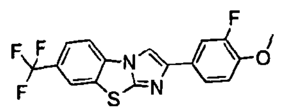 | 59 | 66 |
| 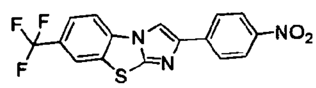 | 30 | 65 |
| 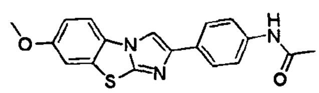 | 44 | 64 |
| 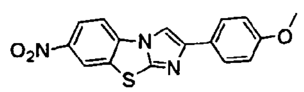 | 29 | 61 |
| 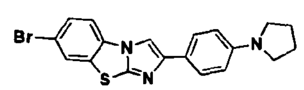 | 60 | 56 |

**Fig. 3.**

| Compound | $K_i$ $A\beta_{40}$ | $K_i$ $A\beta_{42}$ |
|---|---|---|
| 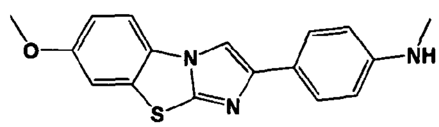 | 3.5 | 5.8 |
| 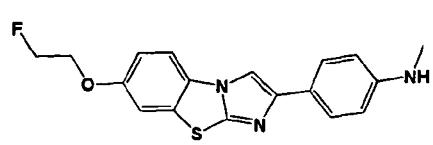 | 2.1 | 4.2 |

**Fig 4.**

| Structure | $A\beta_{40}$ inhibition% | $A\beta_{42}$ inhibition% |
|---|---|---|
| 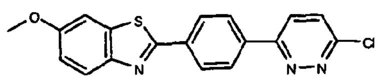 | 78 | 94 |
| 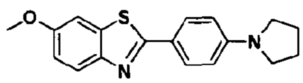 | 76 | 93 |
| 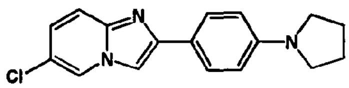 | 64 | 92 |
| 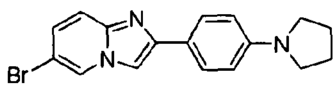 | 77 | 91 |
| 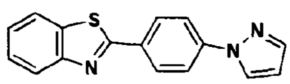 | 71 | 87 |
| 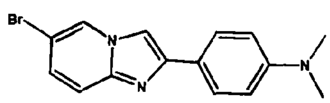 | 56 | 86 |
| 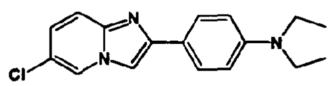 | 57 | 83 |
| 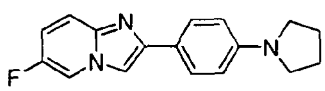 | 57 | 81 |
| 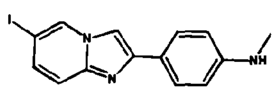 | 52 | 79 |
| 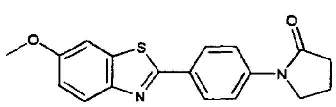 | 58 | 79 |
| 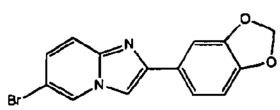 | 52 | 78 |
| 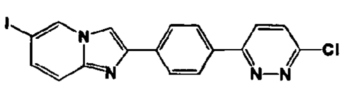 | 73 | 77 |
| 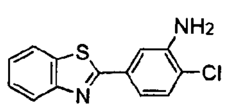 | 43 | 76 |
| 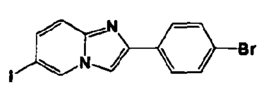 | 40 | 75 |

| | | |
|---|---|---|
| 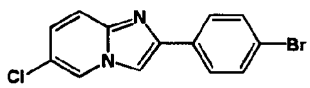 | 36 | 75 |
| 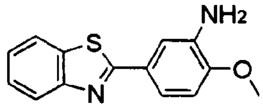 | 50 | 75 |
| 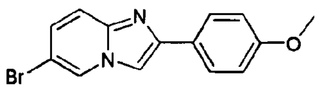 | 43 | 74 |
| 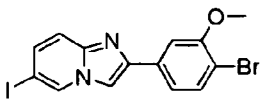 | 35 | 73 |
| 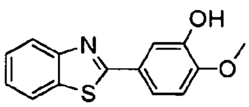 | 11 | 73 |
| 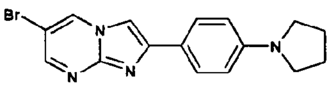 | 24 | 70 |
| 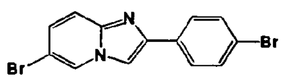 | 47 | 70 |
| 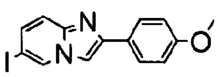 | 38 | 70 |
| 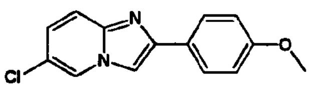 | 30 | 70 |
| 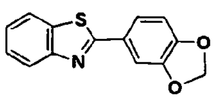 | 36 | 70 |
| 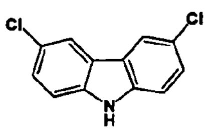 | 11 | 67 |
| 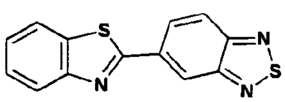 | 36 | 65 |
| 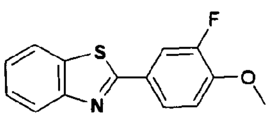 | 22 | 64 |
| 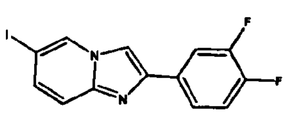 | 23 | 64 |

| | | |
|---|---|---|
| 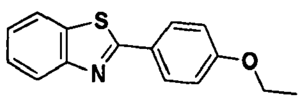 | 32 | 63 |
| 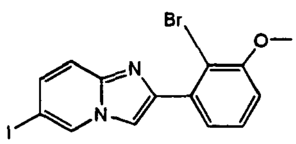 | 29 | 64 |
| 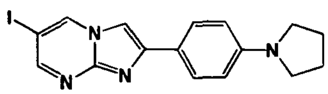 | 47 | 60 |
| 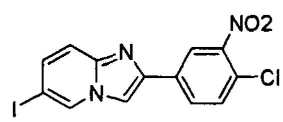 | 17 | 55 |
| 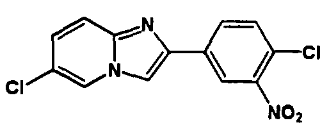 | 0 | 53 |
| 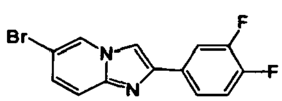 | 5 | 52 |

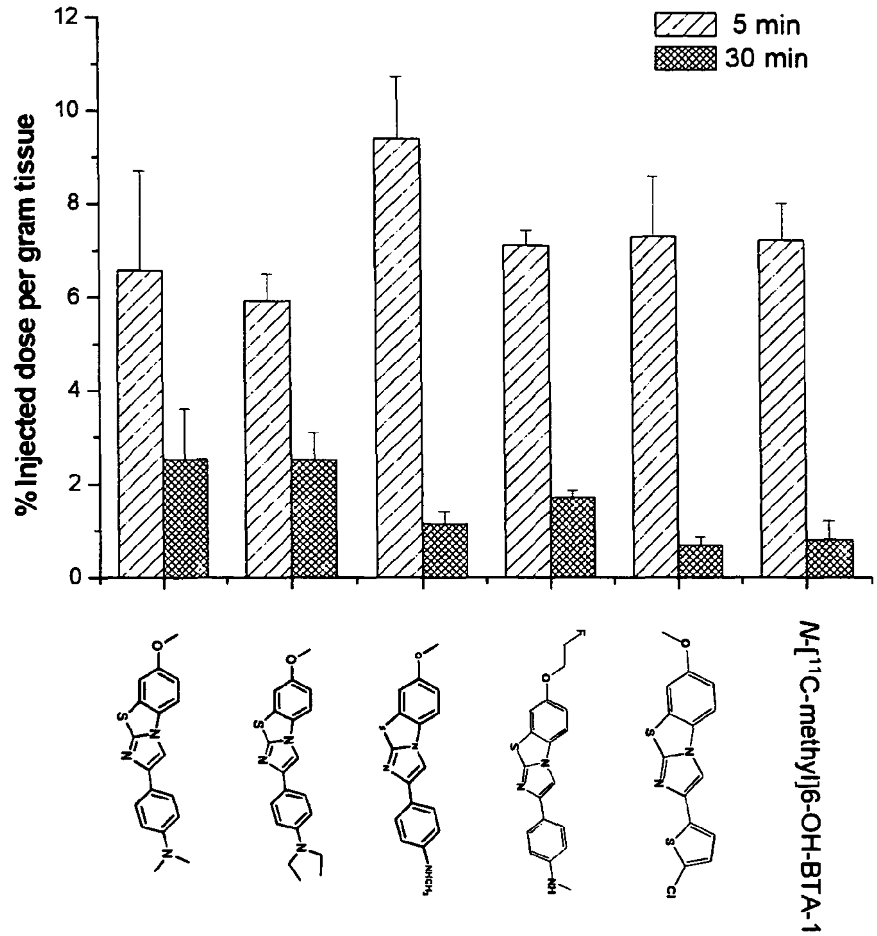


Fig. 5.

Fig. 6.

| Compound | log*P* |
| --- | --- |
| 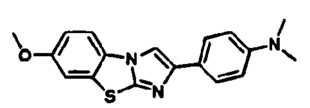 | 1.9 |
| 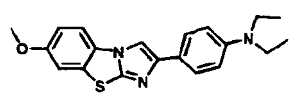 | 2.2 |
| 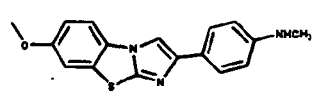 | 1.8 |

| | |
|---|---|
| 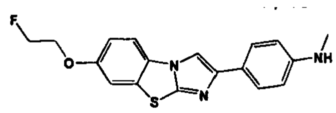 | 1.9 |
| 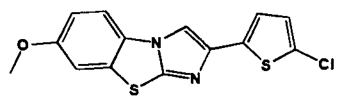 | 2.2 |

Fig. 7.

| | % intact compound | |
|---|---|---|
| Tissue | 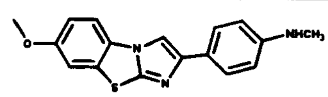 | 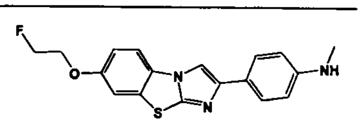 |
| Blood 10 min | 15 ± 5 | 12 ± 3 |
| Blood 30 min | 7 ± 2 | 5± 4 |
| Brain 10 min | 95±2 | 92 ± 4 |
| Brain 30 min | 91±3 | 88 ± 6 |

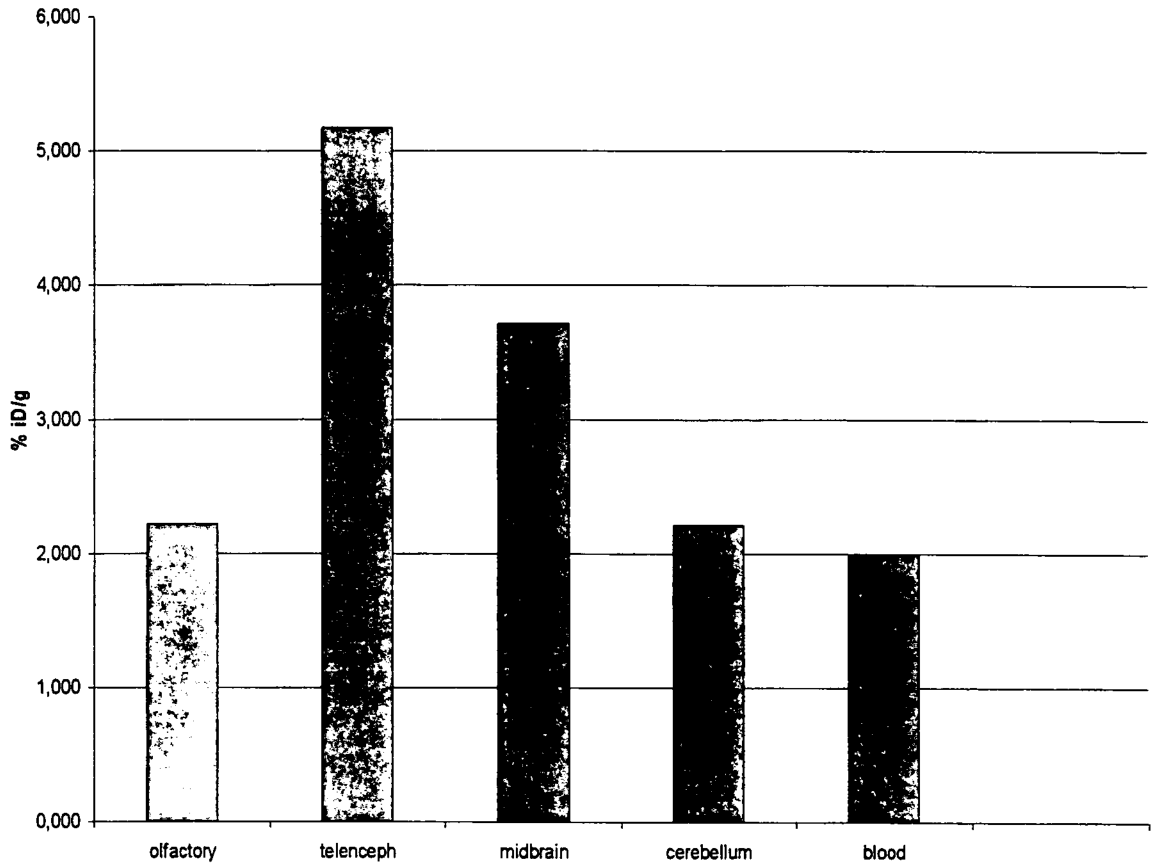
6,000
5,000
4,000
3,000
2,000
1,000
0,000
% iD/g
olfactory
telenceph
midbrain
cerebellum
blood

Fig. 8.

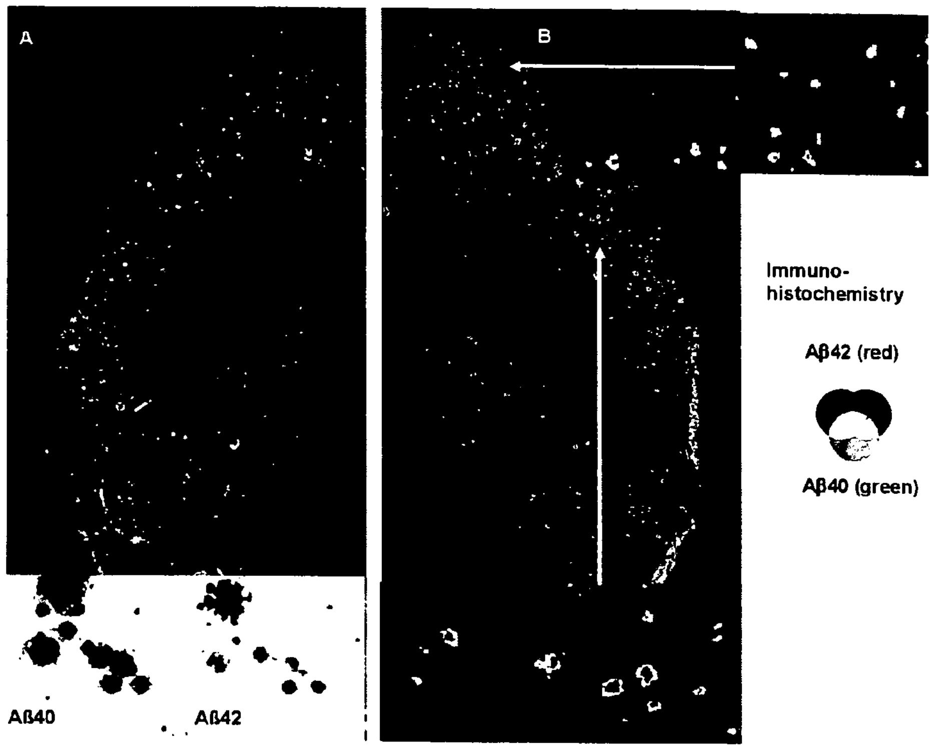


Fig. 9.

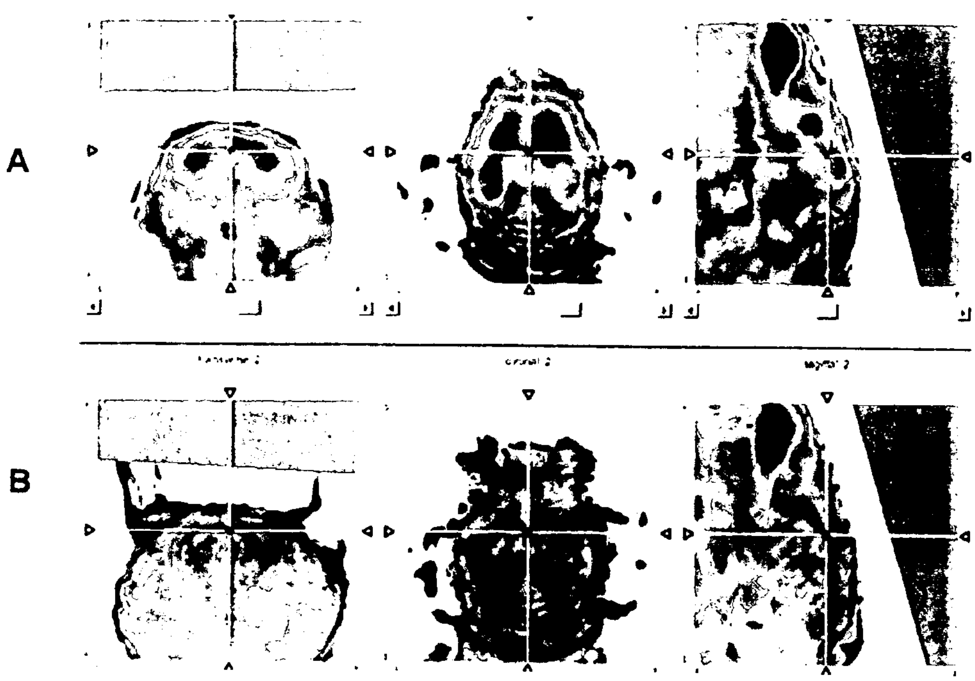


Fig. 10.

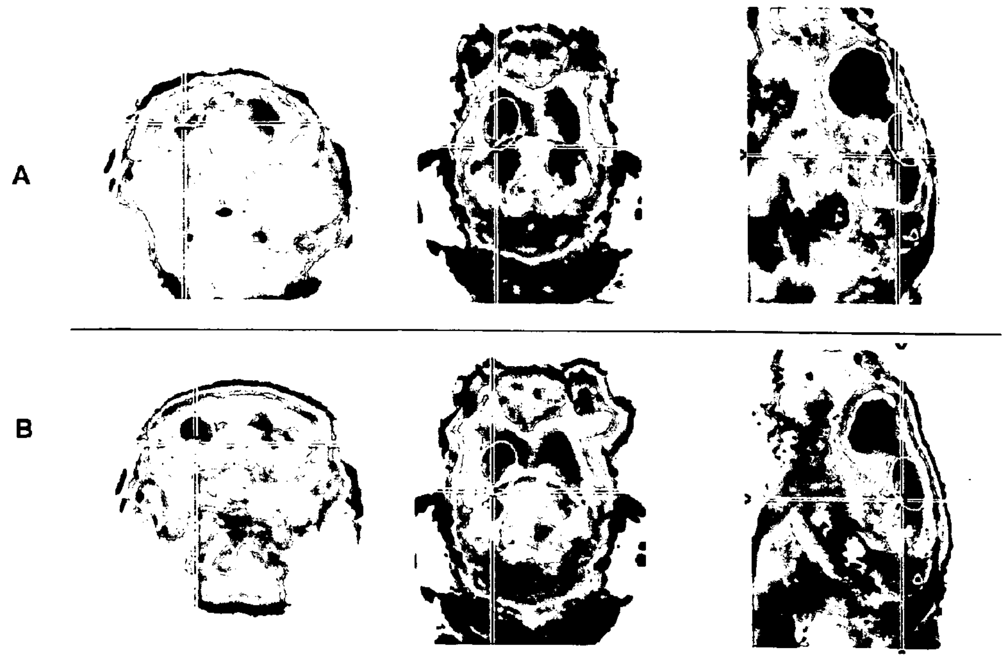


Fig. 11.

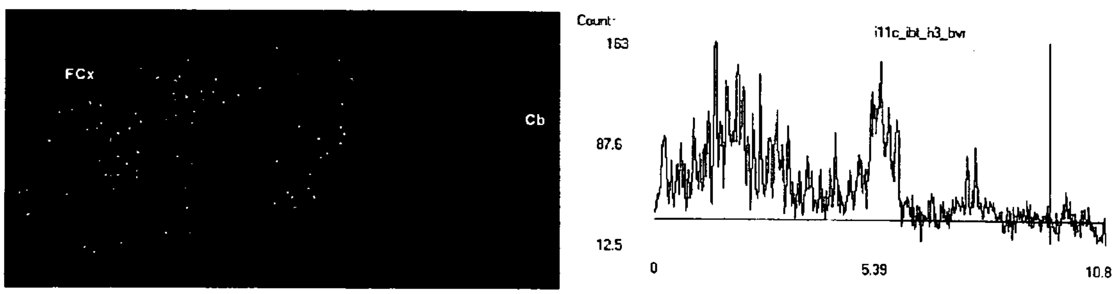


Fig. 12.

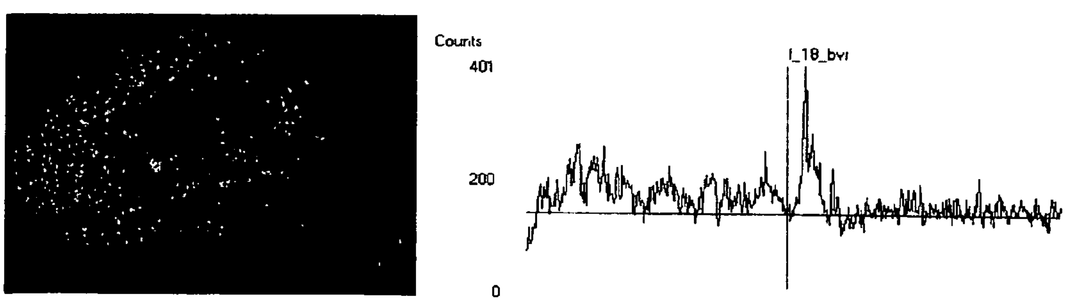


Fig. 13..

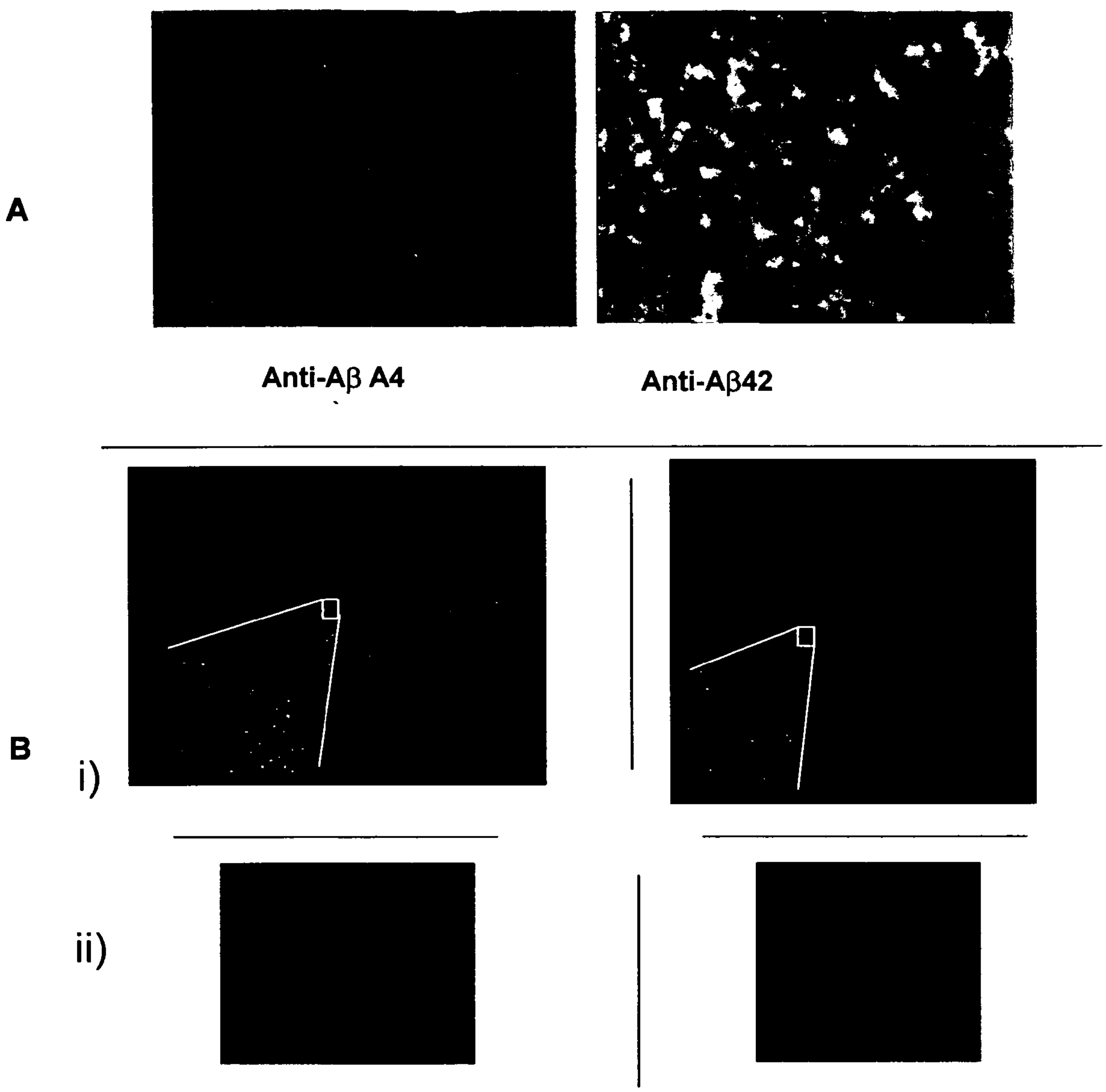


Fig. 14.

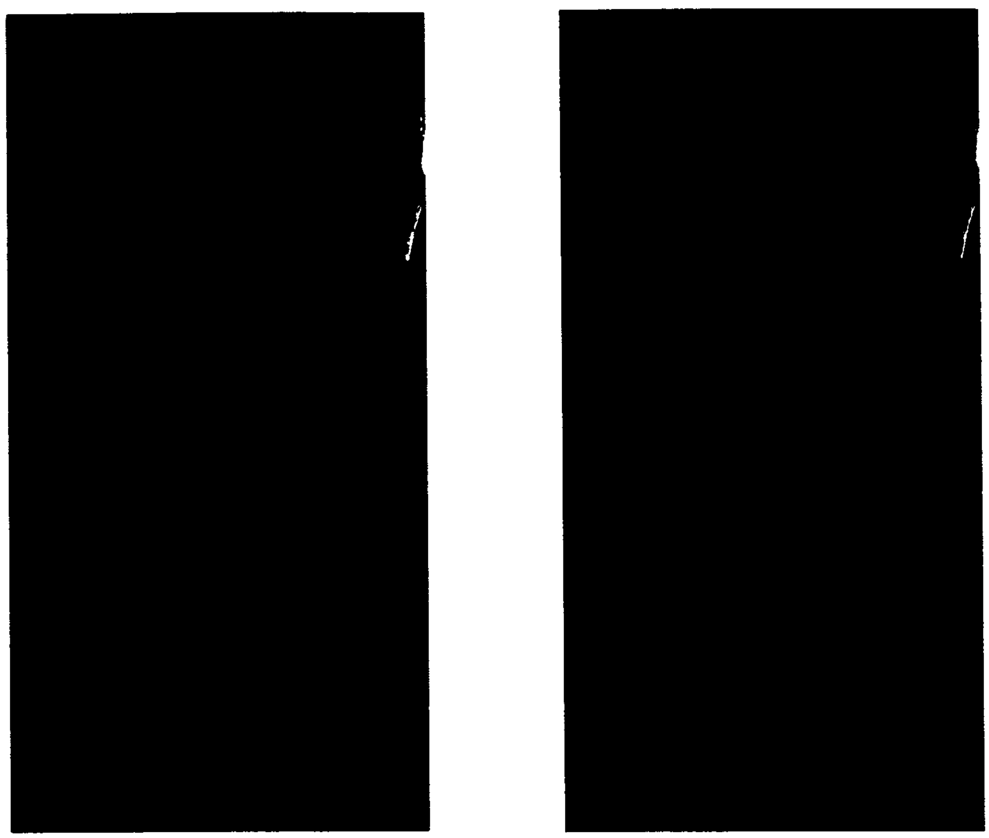

A B

Fig. 15.

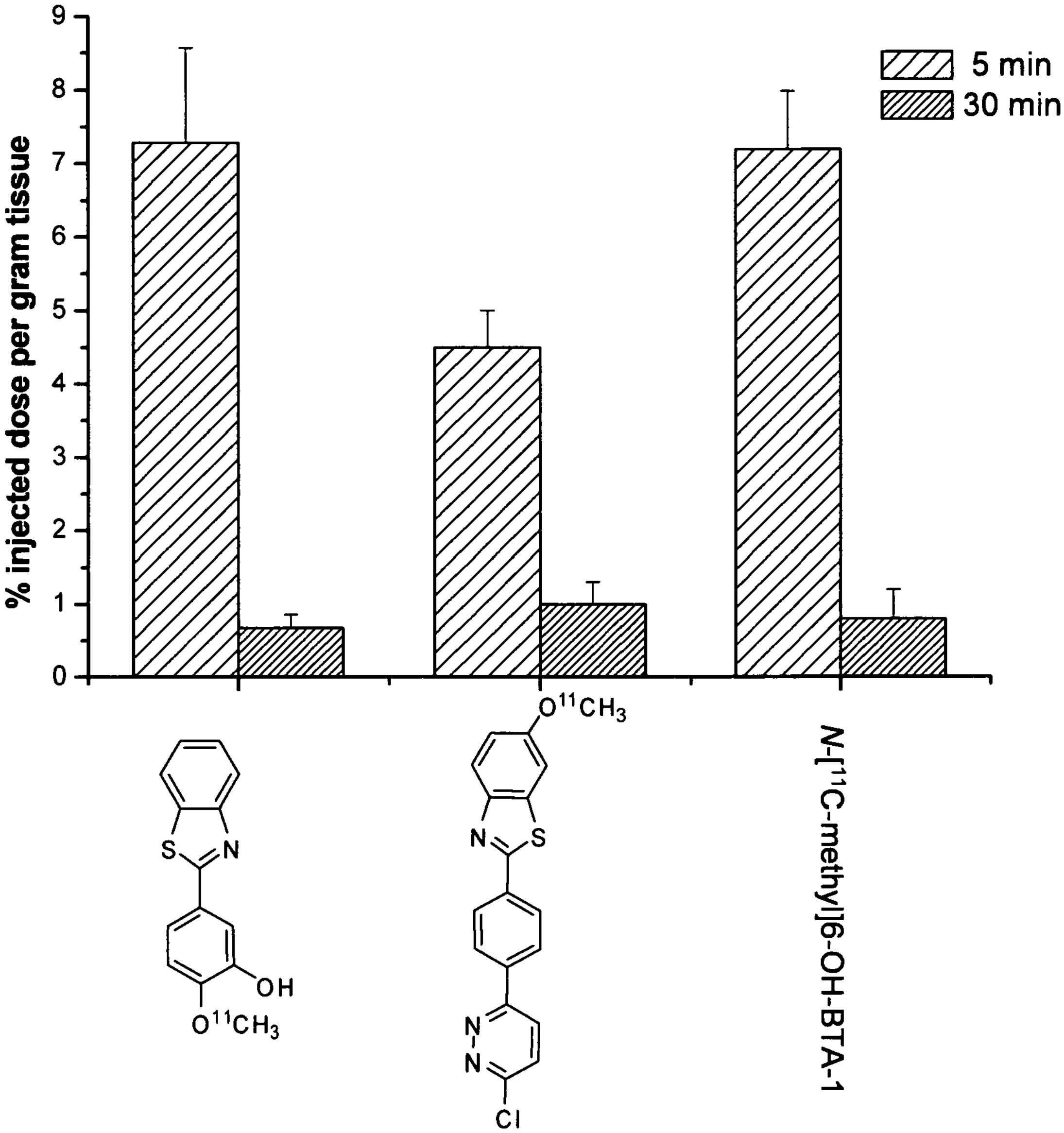
% injected dose per gram tissue
0
1
2
3
4
5
6
7
8
9
5 min
30 min
N-[11C-methyl]6-OH-BTA-1

Fig. 16.

Europäisches Patentamt
European Patent Office
Office européen des brevets

# EUROPEAN SEARCH REPORT

Application Number
EP 09 00 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 2 019 103 A (NIHON MEDIPHYSICS CO LTD [JP]) 28 January 2009 (2009-01-28)<br>* paragraphs [0001], [0228], [0261], [0354], [0358], [0413] *<br>* page 10; table 1-1 *<br>* pages 27-28; table 1-10 *<br>* page 29; table 2-1 *<br>* claims 3-5 *<br>----- | 1,2,11, 12 | INV.<br>A61K51/04<br>C07D471/04<br>C07D277/62<br>C07D513/04<br>C07D487/04<br><br>ADD.<br>A61K101/02 |
| X | WO 2007/148755 A (NIHON MEDIPHYSICS CO LTD [JP]; GE HEALTHCARE LTD [GB]; TANIFUJI SHIGEY) 27 December 2007 (2007-12-27)<br>* abstract * | 1,11,12 | |
| E | -& EP 2 042 501 A (NIHON MEDIPHYSICS CO LTD [JP]; GE HEALTHCARE LTD [GB]) 1 April 2009 (2009-04-01)<br>* paragraphs [0001], [0091], [0103], [0125] *<br>* claims 5-7 *<br>----- | 1,11,12 | |
| X | WO 2007/125988 A (NIHON MEDIPHYSICS CO LTD [JP]; TANIFUJI SHIGEYUKI [JP]; NAKAMURA DAISA) 8 November 2007 (2007-11-08)<br>* abstract * | 1,11,12 | TECHNICAL FIELDS SEARCHED (IPC)<br>A61K<br>C07D |
| E | -& EP 2 022 792 A (NIHON MEDIPHYSICS CO LTD [JP]) 11 February 2009 (2009-02-11)<br>* paragraph [0001] *<br>* page 7; table 1; compounds 1-6,9,11 *<br>* claims 9-12 *<br>----- | 1,11,12 | |
| X | EP 1 944 281 A (UNIV MUENCHEN TECH [DE]) 16 July 2008 (2008-07-16)<br>* page 2; figure 1 *<br>* page 10, line 5 *<br>* claims 7,12,13 *<br>-----<br>-/-- | 1,11,12 | |

~~The present search report has been drawn up for all claims~~

3

| Place of search | Date of completion of the search | Examiner |
|---|---|---|
| The Hague | 17 August 2009 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 00 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2007/002540 A (KUNG HANK F [US]) 4 January 2007 (2007-01-04) * paragraphs [0013], [0043], [0044] * | 1,11,12 | |
| X | WO 2007/033080 A (UNIV EMORY [US]; GOODMAN MARK M [US]; ZENG FANXING [US]) 22 March 2007 (2007-03-22) * page 24; schemes 1 and 2 * * paragraphs [0055], [0056] * * claims 21-28 * | 1,11,12 | |
| X | WO 2008/091195 A (ASTRAZENECA AB [SE]; EDIN MICHAELA [SE]; MALMSTROEM JONAS [SE]; PYRING) 31 July 2008 (2008-07-31) * claims 24-31,58 * | 1,11,12 | |
| X | WO 02/085903 A (KUNG HANK [US]; KUNG MEI-PING [US]; ZHUANG ZHI-PING [US]) 31 October 2002 (2002-10-31) * paragraphs [0017], [0031] - [0033], [0073], [0076] * | 1,11,12 | TECHNICAL FIELDS SEARCHED (IPC) |
| X | EP 1 956 013 A (FUJIFILM RI PHARMA CO LTD [JP]; DAIICHI SANKYO CO LTD [JP]) 13 August 2008 (2008-08-13) * paragraphs [0016], [0070] - [0073] * | 1,11,12 | |
| X | WO 2008/059714 A (NIHON MEDIPHYSICS CO LTD [JP]; TANIFUJI SHIGEYUKI [JP]; NAKAMURA DAISA) 22 May 2008 (2008-05-22) * the whole document * | 1,11,12 | |
| X | WO 2008/065785 A (NIHON MEDIPHYSICS CO LTD [JP]; TANIFUJI SHIGEYUKI [JP]; NAKAMURA DAISA) 5 June 2008 (2008-06-05) * the whole document * | 1,11,12 | |

-/--

~~The present search report has been drawn up for all claims~~

3

| Place of search | Date of completion of the search | Examiner |
|---|---|---|
| The Hague | 17 August 2009 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 00 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
| --- | --- | --- | --- |
| X | ZENG F ET AL: "Synthesis and evaluation of two <18>F-labeled imidazo[1,2-a]pyridine analogues as potential agents for imaging beta-amyloid in Alzheimer's disease"<br>BIOORGANIC & MEDICINAL CHEMISTRY LETTERS, PERGAMON, ELSEVIER SCIENCE, GB,<br>vol. 16, no. 11, 1 June 2006 (2006-06-01), pages 3015-3018, XP025106168<br>ISSN: 0960-894X [retrieved on 2006-06-01]<br>* abstract *<br>* figure 1 * | 1,11,12 | |
| X | WO 2008/118122 A (MOLECULAR NEUROIMAGING LLC [US]) 2 October 2008 (2008-10-02)<br>* page 5, lines 18-21 *<br>* page 5, line 34 - page 6, line 9 *<br>* page 8, lines 16-18 *<br>* page 22; table 3 *<br>* page 24; table 4 *<br>* page 43, lines 20,21 *<br>* examples I,VII,VIII,X *<br>* claims 3,6,8 * | 1-12 | TECHNICAL FIELDS SEARCHED (IPC) |
| X | WO 2007/109120 A (AMBIT BIOSCIENCES CORP [US]; BHAGWAT SHRIPAD [US]; CHAO QI [US]; GROTZ) 27 September 2007 (2007-09-27)<br>* pages 41-46; tables A,B,C * | 1 | |
| X | US 3 928 311 A (FISHER JOHN G ET AL) 23 December 1975 (1975-12-23)<br>* example 4 * | 1 | |
| X | WO 2004/048368 A (PHARMACIA CORP [US]; BECKER DANIEL P [US]; CARROLL JEFFERY N [US]; FOB) 10 June 2004 (2004-06-10)<br>* page 54; compound 42-1 * | 1 | |
| | -/-- | | |

~~The present search report has been drawn up for all claims~~

| Place of search | Date of completion of the search | Examiner |
| --- | --- | --- |
| The Hague | 17 August 2009 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

3

EPO FORM 1503 03.82 (P04C01)

Europäisches Patentamt
European Patent Office
Office européen des brevets

# EUROPEAN SEARCH REPORT

Application Number
EP 09 00 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2006/122178 A1 (COTTAM HOWARD B [US] ET AL) 8 June 2006 (2006-06-08)<br>* table 3 *<br>----- | 1 | |
| X | GB 2 351 081 A (LILLY FORSCHUNG GMBH [DE]) 20 December 2000 (2000-12-20)<br>* page 38; compound 200 *<br>----- | 1 | |
| X | JP 2001 048786 A (YAMANOUCHI PHARMA CO LTD) 20 February 2001 (2001-02-20)<br>* paragraphs [0036],[0037],[0038],[0052]; tables *<br>----- | 1 | |
| X | JP 11 106340 A (SUMITOMO PHARMA) 20 April 1999 (1999-04-20)<br>* page 9; compound 11 *<br>* page 10; compounds 15,16 *<br>* page 11; compounds 17,18 *<br>----- | 1 | |
| X | JP 57 149288 A (YAMANOUCHI PHARMA CO LTD) 14 September 1982 (1982-09-14)<br>* compounds 1-6,9,11-13,15-17,19-22 *<br>* compounds 24-26,28,30-37,39-42,45-48,59-62 *<br>----- | 1 | TECHNICAL FIELDS SEARCHED (IPC) |
| X | US 4 497 817 A (MURASE KIYOSHI [JP] ET AL) 5 February 1985 (1985-02-05)<br>* examples 1-10,12-19,21-25,28,30-32 *<br>* examples 34-40,43,44,46-71,74-77,96-106 *<br>----- | 1 | |
| A | WO 02/14321 A (UNIV CALIFORNIA [US]; COTTAM HOWARD B [US]; LEONI LORENZO M [US]; CARS) 21 February 2002 (2002-02-21)<br>* claims 1,4 *<br>-----<br>-/-- | 2 | |

~~The present search report has been drawn up for all claims~~

3

| Place of search | Date of completion of the search | Examiner |
|---|---|---|
| The Hague | 17 August 2009 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 00 1902

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | EP 0 605 295 A (SYNTHELABO [FR])<br>6 July 1994 (1994-07-06)<br>* claim 3 *<br>* page 8; table *<br>----- | 2 | |

TECHNICAL FIELDS SEARCHED (IPC)

~~The present search report has been drawn up for all claims~~

3

| Place of search | Date of completion of the search | Examiner |
|---|---|---|
| The Hague | 17 August 2009 | Villard, Anne-Laure |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number

EP 09 00 1902

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [ ] As all searchable claims could be searched without effort justifying an additional fee, the Search Division did not invite payment of any additional fee.

- [X] Only part of the further search fees have been paid within the fixed time limit. The present European search report has been drawn up for those parts of the European patent application which relate to the inventions in respect of which search fees have been paid, namely claims:

  3-10; 1,2,11,12 (partially)

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 09 00 1902

The Search Division considers that the present European patent application does not comply with the requirements of unity of invention and relates to several inventions or groups of inventions, namely:

1. claims: 1,2,11,12 (partially)

A tracer for aggregates of amyloid peptides comprising the first structure of claim 1 wherein A=N; Use of this tracer for the preparation of a diagnostic compound for the imaging and quantification of amyloidosis in Alzheimer's disease, type II diabetes, Down's syndrome, Creutzfeldt-Jacob disease; prion mediated diseases, amyloid polyneuropathy and amyloid cardiomyopathy.

---

2. claims: 1,2,11,12 (partially)

A tracer for aggregates of amyloid peptides comprising the first structure of claim 1 wherein A=C; Use of this tracer for the preparation of a diagnostic compound for the imaging and quantification of amyloidosis in Alzheimer's disease, type II diabetes, Down's syndrome, Creutzfeldt-Jacob disease; prion mediated diseases, amyloid polyneuropathy and amyloid cardiomyopathy.

---

3. claims: 1,11,12 (partially)

A tracer for aggregates of amyloid peptides comprising the second structure of claim 1 wherein X=N; Use of this tracer for the preparation of a diagnostic compound for the imaging and quantification of amyloidosis in Alzheimer's disease, type II diabetes, Down's syndrome, Creutzfeldt-Jacob disease; prion mediated diseases, amyloid polyneuropathy and amyloid cardiomyopathy.

---

4. claims: 3-10;1,2,11,12 (partially)

A tracer for aggregates of amyloid peptides comprising the second structure of claim 1 wherein X=S; Method for preparing this tracer according to claim 3; Use of this tracer for the preparation of a diagnostic compound for the imaging and quantification of amyloidosis in Alzheimer's disease, type II diabetes, Down's syndrome, Creutzfeldt-Jacob disease; prion mediated diseases, amyloid polyneuropathy and amyloid cardiomyopathy.

---

5. claims: 1,11,12 (partially)

Europäisches Patentamt
European Patent Office
Office européen des brevets

# LACK OF UNITY OF INVENTION SHEET B

Application Number
EP 09 00 1902

The Search Division considers that the present European patent application does not comply with the requirements of unity of invention and relates to several inventions or groups of inventions, namely:

A tracer for aggregates of amyloid peptides comprising the second structure of claim 1 wherein X=O; Use of this tracer for the preparation of a diagnostic compound for the imaging and quantification of amyloidosis in Alzheimer's disease, type II diabetes, Down's syndrome, Creutzfeldt-Jacob disease; prion mediated diseases, amyloid polyneuropathy and amyloid cardiomyopathy.

---

**ANNEX TO THE EUROPEAN SEARCH REPORT ON EUROPEAN PATENT APPLICATION NO.** EP 09 00 1902

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2009

| Patent document cited in search report | | Publication date | Patent family member(s) | | | Publication date |
|---|---|---|---|---|---|---|
| EP 2019103 | A | 28-01-2009 | AU | 2007252658 | A1 | 29-11-2007 |
| | | | CA | 2653783 | A1 | 29-11-2007 |
| | | | WO | 2007135890 | A1 | 29-11-2007 |
| | | | KR | 20090010987 | A | 30-01-2009 |
| WO 2007148755 | A | 27-12-2007 | AU | 2007261985 | A1 | 27-12-2007 |
| | | | CA | 2655826 | A1 | 27-12-2007 |
| | | | EP | 2042501 | A1 | 01-04-2009 |
| | | | KR | 20090025282 | A | 10-03-2009 |
| EP 2042501 | A | 01-04-2009 | AU | 2007261985 | A1 | 27-12-2007 |
| | | | CA | 2655826 | A1 | 27-12-2007 |
| | | | WO | 2007148755 | A1 | 27-12-2007 |
| | | | KR | 20090025282 | A | 10-03-2009 |
| WO 2007125988 | A | 08-11-2007 | AU | 2007244313 | A1 | 08-11-2007 |
| | | | CA | 2657312 | A1 | 08-11-2007 |
| | | | CN | 101472923 | A | 01-07-2009 |
| | | | EP | 2022792 | A1 | 11-02-2009 |
| | | | KR | 20080112343 | A | 24-12-2008 |
| EP 2022792 | A | 11-02-2009 | AU | 2007244313 | A1 | 08-11-2007 |
| | | | CA | 2657312 | A1 | 08-11-2007 |
| | | | CN | 101472923 | A | 01-07-2009 |
| | | | WO | 2007125988 | A1 | 08-11-2007 |
| | | | KR | 20080112343 | A | 24-12-2008 |
| EP 1944281 | A | 16-07-2008 | NONE | | | |
| WO 2007002540 | A | 04-01-2007 | AU | 2006261917 | A1 | 04-01-2007 |
| | | | CA | 2617319 | A1 | 04-01-2007 |
| | | | EP | 1893245 | A2 | 05-03-2008 |
| | | | JP | 2008546804 | T | 25-12-2008 |
| WO 2007033080 | A | 22-03-2007 | US | 2008219922 | A1 | 11-09-2008 |
| WO 2008091195 | A | 31-07-2008 | NONE | | | |
| WO 02085903 | A | 31-10-2002 | AT | 349446 | T | 15-01-2007 |
| | | | CA | 2444214 | A1 | 31-10-2002 |
| | | | CN | 1620454 | A | 25-05-2005 |
| | | | DE | 60217090 | T2 | 12-07-2007 |
| | | | EP | 1381604 | A2 | 21-01-2004 |
| | | | ES | 2274973 | T3 | 01-06-2007 |
| | | | JP | 2005512945 | T | 12-05-2005 |
| | | | TW | 245764 | B | 21-12-2005 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 09 00 1902

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2009

| Patent document cited in search report | | Publication date | Patent family member(s) | | | Publication date |
|---|---|---|---|---|---|---|
| WO 02085903 | A | | US | 2006051293 | A1 | 09-03-2006 |
| EP 1956013 | A | 13-08-2008 | WO | 2007063946 | A1 | 07-06-2007 |
| | | | US | 2009162283 | A1 | 25-06-2009 |
| WO 2008059714 | A | 22-05-2008 | AU | 2007320581 | A1 | 22-05-2008 |
| WO 2008065785 | A | 05-06-2008 | AU | 2007326707 | A1 | 05-06-2008 |
| WO 2008118122 | A | 02-10-2008 | EP | 2023919 | A2 | 18-02-2009 |
| | | | US | 2007258887 | A1 | 08-11-2007 |
| WO 2007109120 | A | 27-09-2007 | AU | 2007227495 | A1 | 27-09-2007 |
| | | | CA | 2646437 | A1 | 27-09-2007 |
| | | | CN | 101448843 | A | 03-06-2009 |
| | | | EP | 2001892 | A2 | 17-12-2008 |
| | | | KR | 20080108548 | A | 15-12-2008 |
| | | | US | 2007232604 | A1 | 04-10-2007 |
| US 3928311 | A | 23-12-1975 | NONE | | | |
| WO 2004048368 | A | 10-06-2004 | AU | 2003300800 | A1 | 18-06-2004 |
| | | | BR | 0316506 | A | 04-10-2005 |
| | | | CA | 2506796 | A1 | 10-06-2004 |
| | | | EP | 1565459 | A2 | 24-08-2005 |
| | | | JP | 2006513270 | T | 20-04-2006 |
| | | | MX | PA05005474 | A | 25-07-2005 |
| US 2006122178 | A1 | 08-06-2006 | NONE | | | |
| GB 2351081 | A | 20-12-2000 | AU | 5722800 | A | 09-01-2001 |
| | | | WO | 0078726 | A1 | 28-12-2000 |
| JP 2001048786 | A | 20-02-2001 | NONE | | | |
| JP 11106340 | A | 20-04-1999 | NONE | | | |
| JP 57149288 | A | 14-09-1982 | NONE | | | |
| US 4497817 | A | 05-02-1985 | CA | 1158246 | A1 | 06-12-1983 |
| | | | DE | 3030982 | A1 | 12-03-1981 |
| | | | ES | 8106535 | A1 | 01-11-1981 |
| | | | FR | 2463774 | A1 | 27-02-1981 |
| | | | GB | 2056982 | A | 25-03-1981 |
| | | | IT | 1166483 | B | 06-05-1987 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO. EP 09 00 1902

This annex lists the patent family members relating to the patent documents cited in the above-mentioned European search report.
The members are as contained in the European Patent Office EDP file on
The European Patent Office is in no way liable for these particulars which are merely given for the purpose of information.

17-08-2009

| Patent document cited in search report | Publication date | Patent family member(s) | Publication date |
|---|---|---|---|
| WO 0214321 A | 21-02-2002 | AU 9451501 A | 25-02-2002 |
| EP 0605295 A | 06-07-1994 | AU 666790 B2 | 22-02-1996 |
| | | AU 5276293 A | 14-07-1994 |
| | | CA 2112555 A1 | 01-07-1994 |
| | | CN 1096029 A | 07-12-1994 |
| | | CZ 9302918 A3 | 13-07-1994 |
| | | FI 935915 A | 01-07-1994 |
| | | FR 2699921 A1 | 01-07-1994 |
| | | HU 70409 A2 | 30-10-1995 |
| | | IL 108225 A | 15-04-1997 |
| | | JP 6234770 A | 23-08-1994 |
| | | NO 934881 A | 01-07-1994 |
| | | NZ 250579 A | 26-07-1995 |
| | | PL 301680 A1 | 11-07-1994 |
| | | SK 149393 A3 | 08-02-1995 |
| | | US 5418248 A | 23-05-1995 |
| | | ZA 9309760 A | 18-08-1994 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Pearson ; Peers.** *J Physiol.,* 2006, vol. 575, 5-10
- **Dickson ; Vikers.** *Neuroscience,* 2001, vol. 105, 99-107
- **Van Broeck et al.** *Neurodegener Dis.,* 2007, vol. 4, 349-65
- **Yan Y, Wang.** *J Mol Biol.,* 2007, vol. 369, 909-16
- **Kim et al.** *J Neurosci.,* 2007, vol. 27, 627-33
- **Shen ; Kelleher.** *Proc Natl Acad Sci U S A.,* 2007, vol. 104, 403-9
- **Hensley et al.** *Proc.Natl. Acad. Sci. USA,* 1994, vol. 91, 3270-3274
- **Yan et al.** *Nature,* 1996, vol. 382, 685-69115
- **Miklossy et al.** *J.Neuropathol. Exp. Neurol.,* 1999, vol. 58, 803-814
- **Citron et al.** *Nat Med,* 1997, vol. 3, 67-72
- **Mann et al.** *Ann Neurol,* 1996, vol. 40, 149-156
- **Kumar-Singh et al.** *Human mutation,* 2006, vol. 27, 686-695
- **Citron.** *Nat Rev Neurosci.,* 2004, vol. 5, 677-85
- **Wilbur.** *Bioconjug Chem.,* 1992, vol. 3, 433-70
- **Maziere et al.** Radioionidation Reactions for Pharmaceuticals. Springer Verlag
- **Henriksen et al.** *J Med Chem,* 2007, vol. 50, 1087-1089